# EUROPEAN PATENT APPLICATION

(11) **EP 4 445 820 A1**
(43) Date of publication of application: **16.10.2024**
(21) Application number: 22891183.0
(22) Date of filing: 30.11.2022
(51) Int. Cl.: A47L 23/20, A47L 23/18, A47B 61/04, F26B 21/00, F26B 7/00, F26B 3/02, F26B 9/00, A61L 2/07

(54) **SHOE CARE APPARATUS**

(30) Priority: 07.12.2021 KR 20210173823; 17.12.2021 KR 20210182035
(71) Applicant: LG Electronics Inc., Seoul 07336 (KR)
(72) Inventor: CHOI, Kyoung Min, Seoul 08592 (KR); JANG, Ju Hyuck, Seoul 08592 (KR); LEE, Young Jae, Seoul 08592 (KR); KIM, Ji Hun, Seoul 08592 (KR)
(74) Representative: Schornack, Oliver
(86) International application number: PCT/KR2022/019213
(87) International publication number: WO 2023/106719

(57) **Abstract**

A shoes care device according to an embodiment includes an inner cabinet and a module housing. A blowing part, a heating part, and a dehumidifying part are jointly accommodated in a module chamber inside a module housing, and the module housing is detachably coupled to a lower side of the inner cabinet. A shoes care device having a structure in which condensed water generated while passing through the blowing part, the heating part, and/or the dehumidifying part can be prevented from being leaked, and it is advantageous for leakage management, and the module housing and a component accommodated therein are easily managed and replaced.

## Description

### [Technical Field]

The present invention relates to a shoes care device, and more particular, to a shoes care device which treats shoes by air circulation.

### [Background Art]

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air.

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

### [Disclosure]

### [Technical Problem]

An object to be solved by the present invention is to provide a shoes care device having a circulated air current structure in which air inside an inner cabinet in which shoes are placed is dehumidified by using a dehumidifying part, and the dehumidified air can be supplied to the inside of the inner cabinet, and having a means capable of regenerating the dehumidifying part, which has a structure of easily preventing water leakage and easily managing a means for drying the air inside the inner cabinet and regenerating the dehumidifying part.

An object to be solved by the present invention is to provide a shoes care device having a structure of not invading another space of a machine room when the means for drying the air inside the inner cabinet and regenerating the dehumidifying part is coupled to or separated from the inner cabinet.

An object to be solved by the present invention is to provide a shoes care device a structure of including a single module housing jointly accommodating the means for drying the air inside the inner cabinet and regenerating the dehumidifying part, stably assembling the module housing and the inner cabinet, and preventing a gap between the module housing and the inner cabinet.

An object to be solved by the present inv3ention is to provide a shoes care device having a structure in which the module housing and the inner cabinet can be stably and easily coupled and separated.

An object to be solved by the present invention is to provide a shoes care device having a structure in which the module housing can be easily coupled to the inner cabinet, and the air can be easily circulated inside the inner cabinet and inside the module housing.

An object to be solved by the present invention is to provide a shoes care device having a structure in which a vertical height of the module housing can be minimized, and spaces of the inner cabinet and/or the machine room can be secured.

An object to be solved by the present invention is to provide a shoes care device having a structure in which a blowing part is placed, when condensed water is generated inside the module housing, the condensed water can be easily discharged to the outside of the module housing, and prevented from remaining inside the blowing part.

An object to be solved by the present invention is to provide a shoes care device having a structure in which when the dehumidifying part is placed inside the module housing, the air can smoothly pass the dehumidifying part and the condensed water generated while passing through the dehumidifying part moves along a bottom surface inside the module housing to be discharged to the outside of the module housing.

### [Technical Solution]

A shoes care device described in this application may be configured to include an inner cabinet, a module housing, a sump, a blowing part, a heating part, a dehumidifying part, a drying flow path, and a regeneration path.

The inner cabinet has an accommodation space accommodating shoes.

The module housing may be configured to be coupled to a lower side of the inner cabinet, and to have a module chamber communicating with the accommodation space therein.

The sump may be configured to be provided at the lower side of the module housing and to accommodate condensed water.

The blowing part may be configured to be accommodated in the module chamber, and to blow air of the module chamber.

The heating part may be configured to be accommodated in the module chamber, and to heat the air of the module chamber.

The dehumidifying part may be configured to be accommodated in the module chamber, and to dehumidify the air of the module chamber.

The drying flow path may form a path so as for the air of the module chamber passing through the dehumidifying part to be re-introduced into the accommodation space.

The regeneration path may form a path so as for the air of the module chamber passing through the dehumidifying part to move to the sump.

The module housing may be detachably coupled to the lower side of the inner cabinet.

The module housing may be coupled to the lower side of the inner cabinet while sliding in a horizontal direction.

The inner cabinet may be configured to include a sliding guide.

The sliding guide may be formed in a first direction which is a horizontal direction.

The module housing may be configured to include a module case, a module cover, and a slider.

The module case may be configured to form the module chamber, and have a module opening opened upward, and have an area on a plan view, which is equal to or larger than an area of the module chamber.

The module cover may cover the module opening at the upper side of the blowing part, the heating part, and the dehumidifying part, and may be coupled to the module case, and may have an outlet through which the air of the accommodation space is suctioned to the module chamber, which is formed to penetrate.

The slider may be configured to be fixed to the module case or the module cover, and to slidably move in a forming direction of the sliding guide.

The sliding guide may be configured to include a lower rail and an upper rail.

The lower rail may be configured to be parallel to the first direction or inclined upward.

The upper rail may be configured to be positioned at the upper side of the lower rail, and to be inclined downward in the first direction.

The slider may be configured to include a lower slider and an upper slider.

The lower slider may be configured to contact the upper side of the lower rail and to be parallel to the lower rail.

The upper slider may be configured to be parallel to the upper rail, and to contact the lower side of the upper rail when the module housing moves at a frontmost portion of the first direction.

The shoes care device may be configured to include a door.

The inner cabinet may be configured to include a main opening of which a front side of the first direction is opened, and the door may be configured to open/close the main opening.

A pair of sliding guides may be provided, and formed to be symmetric to both left and right sides of the inner cabinet based on the first direction.

A pair of sliders may be provided, and formed to be symmetric to both left and right sides of the module housing based on the first direction.

The outlet may be formed in a second direction which is orthogonal to the first direction and which is a horizontal direction at the front of the module cover based on the first direction.

The upper surface of the module cover is inclined downward in the first direction.

The inner cabinet may be configured to include a lower opening of which a lower portion is opened.

When the module housing is coupled to the inner cabinet, the module cover may be configured to cover the lower opening.

The module cover may be coupled to a lower end of the inner cabinet to slidably move in the horizontal direction.

In the shoes care device, two or more inner cabinets, module housings, blowing parts, heating parts, and dehumidifying parts may be each provided.

In the shoes care device, two or more inner cabinets may be configured integrally with each other while being placed vertically.

The inner cabinet may be configured to include a first inner side plate, a second inner side plate, a first sliding guide, a second sliding guide, and a lower opening.

The first inner side plate may be positioned at any one based on a reference plane which is parallel to the first direction which is the horizontal direction, and which is a vertical surface.

The second inner side plate may be positioned at an opposite side to the first inner side plate based on the reference plane.

The first sliding guide may be formed in the first direction at a lower end of the first inner side plate.

The second sliding guide may be formed in the first direction at a lower end of the second inner side plate.

The lower opening may form a hole opened between the first sliding guide and the second sliding guide.

The module cover may be configured to include a first slider and a second slider.

The first slider may be coupled to the first sliding guide to be slidably movable.

The second slider may be coupled to the second sliding guide to be slidably movable.

The inner cabinet may be configured to include a front frame and a fixing guide.

When the first direction is a forward direction, the front frame may be configured to connect a front of the first inner side plate and a front of the second inner side plate to each other.

The fixing guide may be formed on a back surface of the front frame in a second direction which is orthogonal to the first direction and which is the horizontal direction.

The module cover may be configured to include a fixation rib formed in the second direction.

The fixing guide and the fixation rib may be configured to be engaged with each other.

The first sliding guide and the second sliding guide may be symmetric to each other based on the reference plane.

The first slider and the second slider may be symmetric to each other based on the reference plane.

The outlet through which the air of the accommodation space is suctioned to the module chamber may be formed to penetrate at the front of the first direction in the module cover.

The shoes care device may be configured to include a dry air duct.

The dry air duct may connect the module housing and the inner cabinet to be in communication with each other at a rear of the first direction, and form the entirety or a part of the drying flow path.

The module housing may be configured to include a dry air outlet opened at a rear side of the module housing based on the first direction.

The dry air duct may be closely coupled to the module housing and the inner cabinet while covering the dry air outlet.

The module housing may be configured to include a steam inlet provided to be in communication with the accommodation space at the rear of the first direction.

The shoes care device may be configured to include steam generator and a steam separator.

The steam generator may be configured to be provided at the lower side of the module housing, and to generate a steam.

The steam separator may be coupled to the module housing at the rear of the first direction.

The steam separator may be configured to include a separating base coupled to the module housing at the rear of the first direction, and forming an internal space which is in communication with the seam inlet, a separating inlet formed at one side of the separating base and connected to the steam generator, and a separating outlet formed at the lower side of the separating base, and discharging condensed water condensed inside the separating base.

The module chamber may be configured to include a first module chamber accommodating the blowing part, a second module chamber accommodating the heating part, and a third module chamber accommodating the dehumidifying part.

The first module chamber, the second module chamber, and the third module chamber may be formed at different locations on a plan view.

The air of the module chamber may move to the first module chamber, the second module chamber, and the third module chamber sequentially.

The module chamber may be configured to include a suction module chamber. The outlet may be formed to penetrate at the upper side of the suction module chamber.

The module chamber may be configured to include a wet air outlet.

The dry air outlet may be opened so as for the air of the third module chamber to be discharged, and connected to the accommodation space.

The dry air outlet may be opened so as for the air of the third module chamber to be discharged, and connected to the accommodation space.

When a direction in which the module housing is coupled at the lower side of the inner cabinet is the first direction, the suction module chamber, the first module chamber, the third module chamber, and the dry air outlet may be placed in order toward the rear from the front based on the first direction.

A bottom surface of the suction module chamber may be inclined downward toward the first module chamber.

The shoes care device may be configured to include a damper and a condenser.

The damper may close the dry air outlet or the wet air outlet.

The condenser may be connected to be in communication between the wet air outlet and the sump, forming the entirety or a part of the regeneration path, and coupled to an outer surface of the inner cabinet.

The blowing part may be spaced apart from a bottom of the first module chamber so that the air is introduced into the inside of the blowing part from the lower side of the first module chamber inside the first module chamber.

The dehumidifying part may be spaced apart from the bottom of the third module chamber so that the air moves to the lower side by penetrating the dehumidifying part from the upper side of the third module chamber inside the third module chamber.

### [Advantageous Effects]

In the shoes care device according to the embodiment of the present invention, a blowing part, a heating part, and a dehumidifying part are jointly accommodated in a module chamber inside a module housing, and the module housing is detachably coupled to a lower side of the inner cabinet. The air inside the inner cabinet moves to the module chamber to the module housing, and the blowing part, the heating part, and the dehumidifying part which are a main means for drying the air inside the inner cabinet and a main means for regenerating the dehumidifying part are jointly positioned in the module chamber of the module housing. The blowing part, the heating part, and the dehumidifying part are positioned very close to each other. The bottom parts of the module housing may be configured integrally, and the bottom parts may not be assembled to each other, and any gap may not be formed on the bottom. Therefore, a shoes care device can be provided, which has a structure in which the condensed water inside the inner cabinet may be introduced into the module chamber inside the module housing, and the condensed water generated while passing through the blowing part, the heating part, and/or the dehumidifying part can be effectively prevented form being leaked from the module housing, and it is advantageous for leakage management. Further, a shoes care device having a structure in which the module housing and a component accommodated therein are easily managed and replaced can be provided.

In the shoes care device according to the embodiment of the present invention, the module housing is coupled to the lower side of the inner cabinet while sliding in a horizontal direction. Therefore, when the module housing is coupled to or separated from the lower side of the inner cabinet, it is not necessary to invade another space of the machine room, and a vertical height of the machine room can be prevented from increasing.

In the shoes care device according to the embodiment of the present invention, the inner cabinet includes the sliding guide and the module housing includes the slider. In addition, the sliding guide includes the lower rail and the upper rail, and the slider includes the lower slider and the upper slider. While the slider moves in the forming direction of the sliding guide, the module housing is coupled to the inner cabinet, and when the module housing moves to the frontmost portion of the first direction, the upper rail and the upper slider contact each other, so the upper rail serves as a stopper. Therefore, while the module housing moves in the first direction, the module housing and the inner cabinet are stably assembled, and a vertical gap and a forward-backward gap between the module housing and the inner cabinet are effectively prevented.

In the shoes care device according to the embodiment of the present invention, each of the sliding guide and the slider is provided as a pair, and formed to be symmetric to each other at both left and right sides of the inner cabinet based on the first direction. Therefore, the inner cabinet of the module housing can be stably and easily coupled and separated.

In the shoes care device according to the embodiment of the present invention, the outlet through which the air of the accommodation space is suctioned is formed to penetrate in the module cover forming the module housing, the inner cabinet includes the lower opening of which a lower portion is opened, and when the module housing is coupled to the inner cabinet, the module cover is configured to cover the lower opening. That is, the module cover of the module housing forms the bottom surface of the inner cabinet, and while the inner cabinet and the module housing are stably coupled to each other, a shoes care device having a simple structure may be formed.

Further, the module cover of the module housing forms the bottom surface of the inner cabinet, so even when a comparative complicated structure is required on the lower surface of the of the module cover, the module cover can be easily formed, and the bottom surface of the inner cabinet may be easily formed.

The shoes care device according to the embodiment includes a dry air duct connecting the module housing and the inner cabinet to be in communication with each other. In the module cover, the outlet is formed at the front of the first direction, and the dry air duct connects the module housing and the inner cabinet to be in communication with each other at the rear of the first direction. Therefore, after the module housing is first coupled to the inner cabinet, the dry air duct can be coupled to the inner cabinet and the module housing, and a shoes care device having a structure in which the air can be circulated inside the inner cabinet and inside the module housing can be easily formed.

In the shoes care device according to the embodiment of the present invention, the module chamber of the module housing includes the first module chamber accommodating the blowing part, the second module chamber accommodating the heating part, and the third module chamber accommodating the dehumidifying part. The first module chamber, the second module chamber, and the third module chamber are formed at different locations on the plan view. Therefore, a shoes care device having a structure in which the vertical height of the module housing can be minimized, and the spaces of the inner cabinet and/or the machine room can be secured can be formed.

In the shoes care device according to the embodiment of the present invention, the blowing part is spaced apart from the bottom of the first module chamber so that the air is introduced into the inside the blowing part from the lower side of the first module chamber inside the first module chamber. Therefore, even though the condensed water is generated inside the first module chamber, the condensed water can move along the bottom surface of the first module chamber, and be discharged to the outside of the module housing, and prevented or minimized from remaining inside the blowing part.

In the shoes care device according to the embodiment of the present invention, the dehumidifying part is spaced apart from the bottom of the third module chamber so that the air inside the third module chamber moves from the upper side of the third module chamber to the lower side through the dehumidifying part. Therefore, the air can smoothly pass through the dehumidifying part inside the third module chamber, and the condensed water generated while passing through the dehumidifying part can move along the bottom surface of the third module chamber and be discharged to the outside of the module housing.

### [Description of Drawings]

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together.
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12a is an exploded perspective view illustrating the condenser according to one embodiment of the present invention.
FIG. 12B is a view illustrating an inner state of the condenser of FIG. 12a.
FIG. 12C is a front view illustrating the condenser of FIG. 12b.
FIG. 13 is a cross-sectional view illustrating a first module chamber of a module housing in the shoes care device according to one embodiment of the present invention.
FIG. 14 is a perspective view illustrating a dehumidifying part and a dehumidifying material housing according to one embodiment of the present invention.
FIG. 15 is a bottom perspective view illustrating a module cover according to one embodiment of the present invention.
FIG. 16 is a cross-sectional view illustrating a part of a third module chamber of the module housing in the shoes care device according to the present invention.
FIG. 17 is a perspective view illustrating a steam separator according to one embodiment of the present invention.
FIG. 18a is a cross-sectional view illustrating a part of a separating inlet of a steam separator in the shoes care device according to one embodiment of the present invention.
FIG. 18b is a cross-sectional view illustrating a part of a separating connector of the steam separator in the shoes care device according to one embodiment of the present invention.
FIG. 19 is a perspective view illustrating a state in which the inner cabinet and the module housing are coupled according to one embodiment of the present invention.
FIG. 20 is a perspective view illustrating the inner cabinet illustrated in FIG. 19.
FIG. 21 is a perspective view of the inner cabinet illustrated in FIG. 20 when viewed from the back.
FIG. 22a is a front view illustrating the inner cabinet illustrated in FIG. 20, and FIG. 22a is a rear view illustrating the inner cabinet illustrated in FIG. 20.
FIGS. 23a and 23b are cross-sectional perspective views illustrating the inner cabinet illustrated in FIG. 20.
FIGS. 24a and 24b are side cross-sectional views illustrating the inner cabinet illustrated in FIG. 20.
FIG. 25a is a cross-sectional view schematically illustrating a mold used in manufacturing the inner cabinet according to one embodiment of the present invention, and FIG. 25b is a schematic view illustrating a state in which the mold of FIG. 25a is closed.
FIG. 26 is a side view illustrating a state in which the inner cabinet, the module housing, and a dry air duct are separated from each other in the shoes care device according to one embodiment of the present invention.
FIG. 27a is a side view illustrating the module cover according to one embodiment of the present invention.
FIG. 27b is a cross-sectional view illustrating the inner cabinet according to one embodiment of the present invention.
FIG. 28a is a cross-sectional perspective view of a part of the shores care device before the coupling of the module housing and the inner cabinet is completed, and FIG. 28b is a cross-sectional perspective view of a part of the shores care device where the coupling of the module housing and the inner cabinet is completed.
FIG. 29 is a cross-sectional view illustrating a state in which the inner cabinet and the module housing are coupled to each other in the shores care device according to one embodiment of the present invention.
FIG. 30a is a perspective view illustrating a lower side of the inner cabinet and a part of a first wall, and FIG. 30b is a rear perspective view illustrating the lower side of the inner cabinet and a part of the first wall.

### [Modes for the Invention]

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

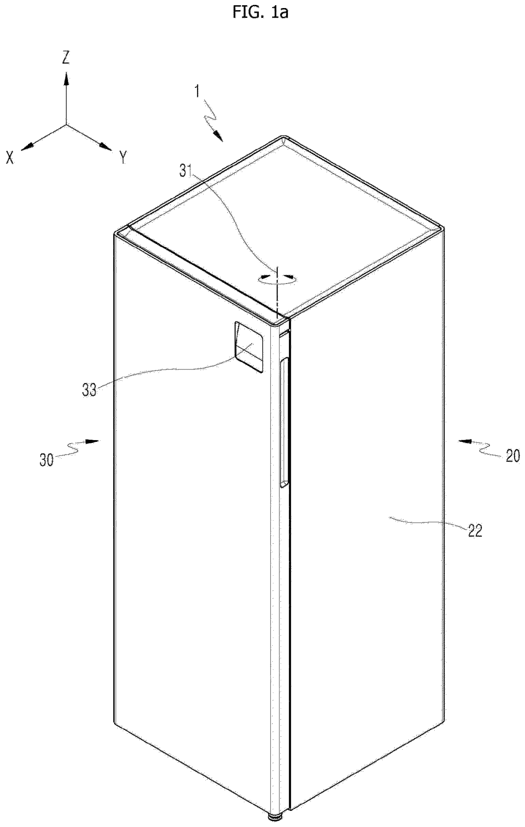

FIG. 1a is a perspective view illustrating a shoes care device 1 according to one embodiment of the present invention.

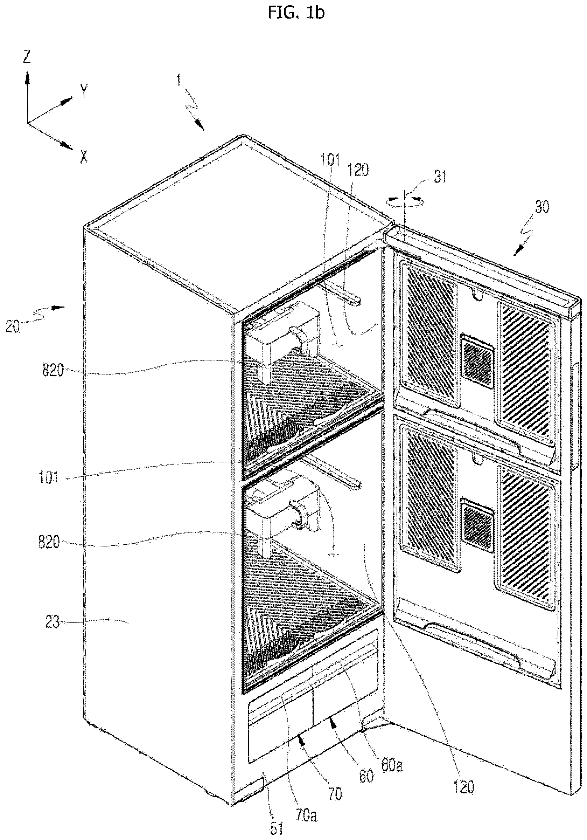

FIG. 1b is a perspective view of the shoes care device of FIG. 1a, when viewed from another direction, illustrating a state in which a door is opened.

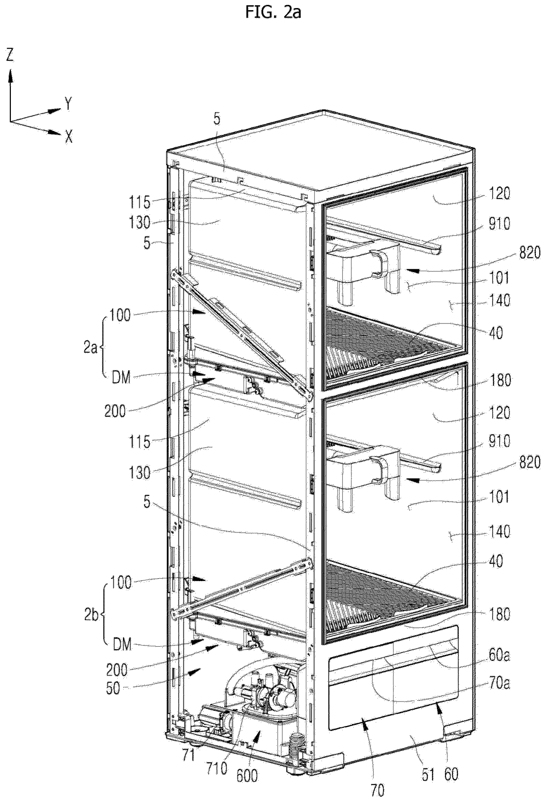

FIG. 2a is a perspective view illustrating a state in which a part of the door and an outer cabinet are removed from the shoes care device of FIG. 1b.

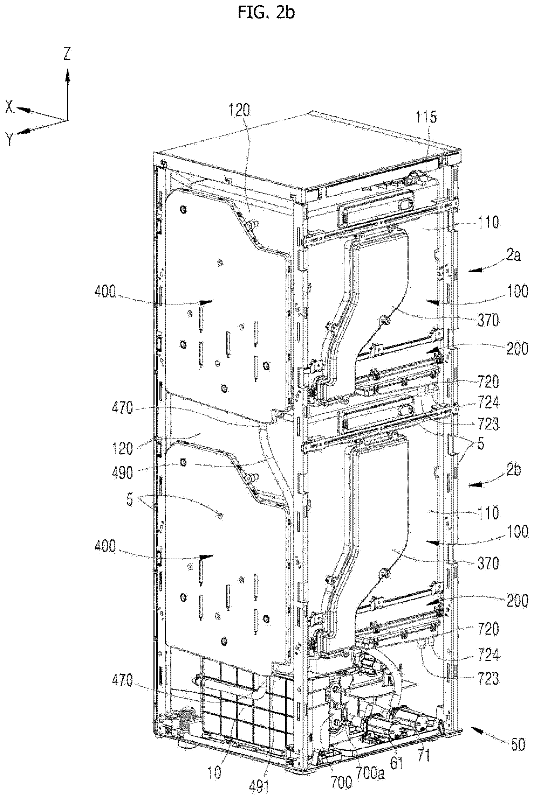

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

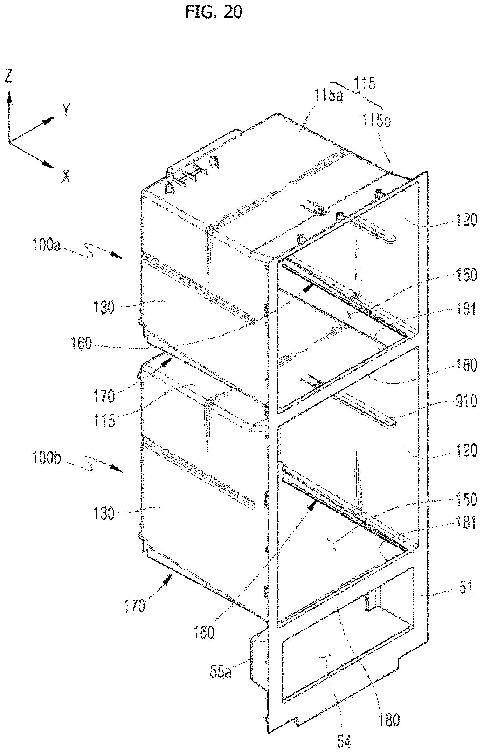

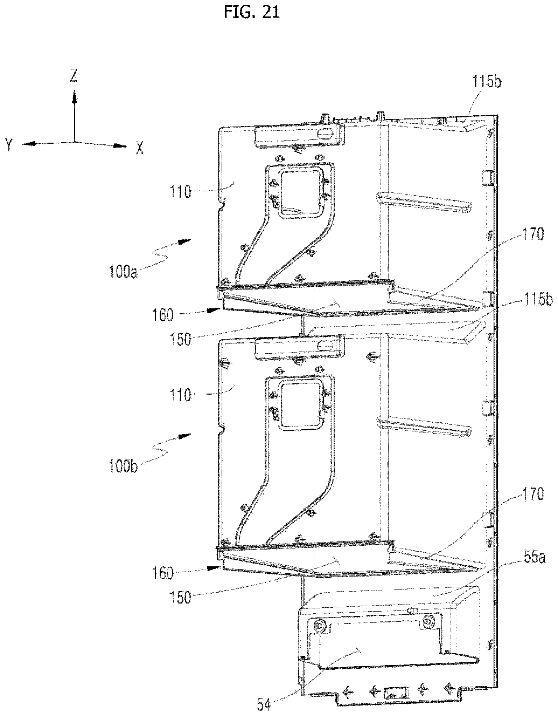

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIG. 20 and FIG. 21).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

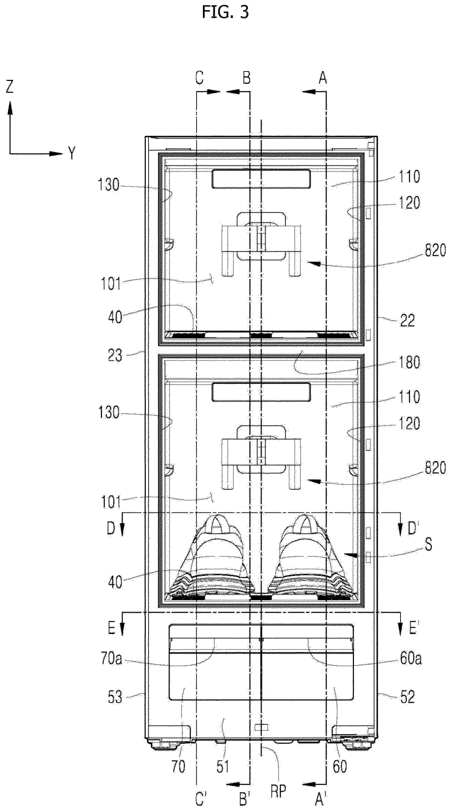

FIG. 3 is a front view illustrating a state in which a door is removed from the shoes care device illustrated in FIG. 1b. FIG. 3 illustrates shoes accommodated in an inner cabinet together.

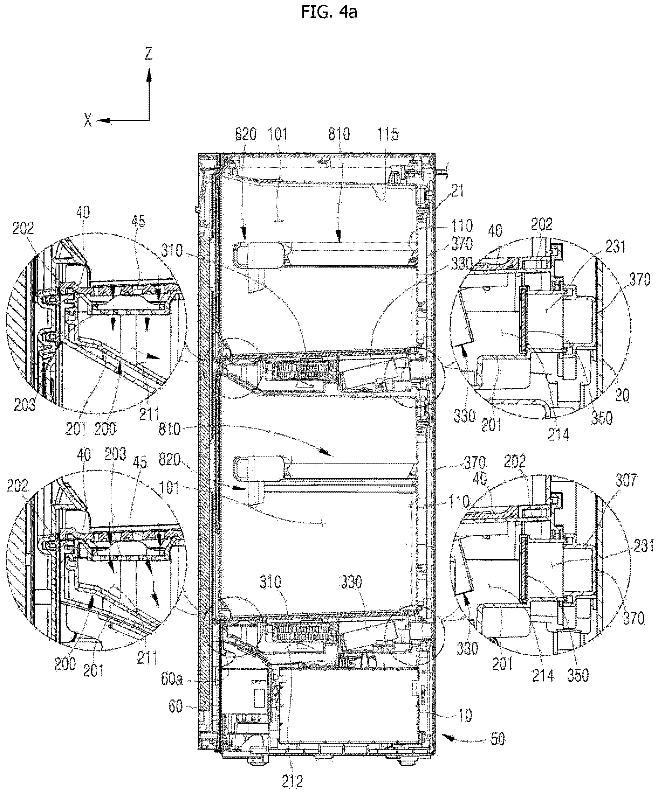

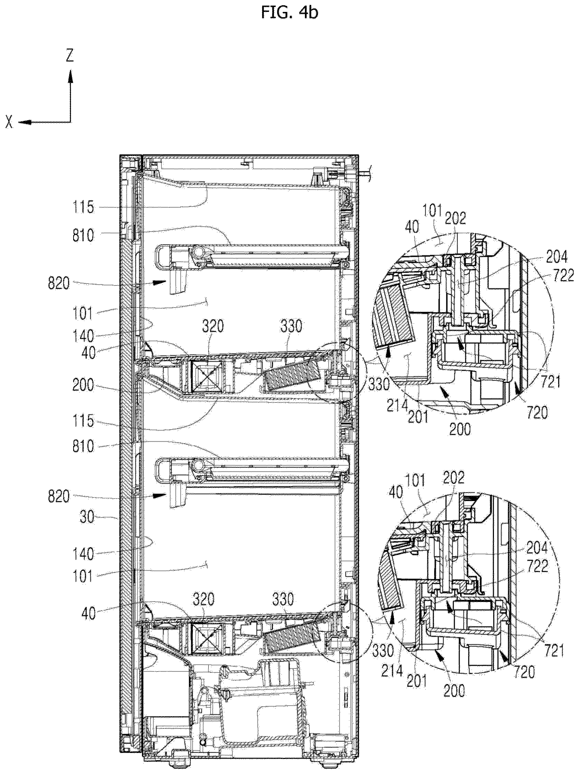

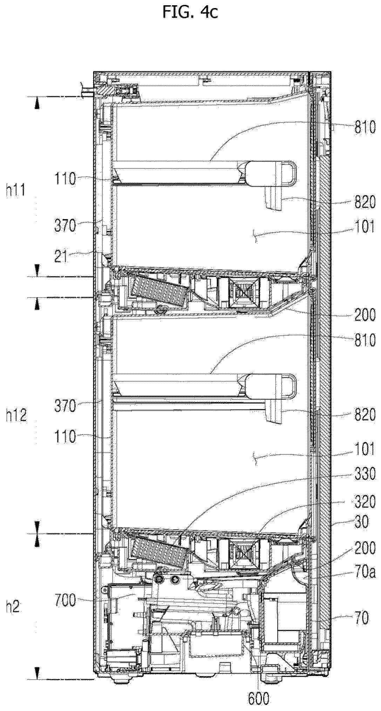

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include an outlet 203 and a nozzle 820.

The management devices 2a and 2b may include the module housing 200 forming a connection path F10. The module housing 200 forms all or part of the connection path F10

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

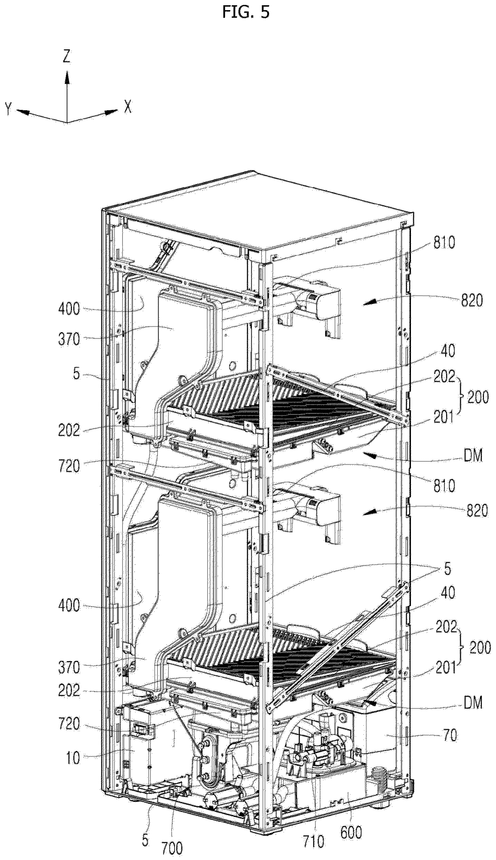

FIG. 5 is a perspective view illustrating a state in which the door 30, the outer cabinet 20, and the inner cabinet 100 are removed from the shoes care device 1 according to one embodiment of the present invention.

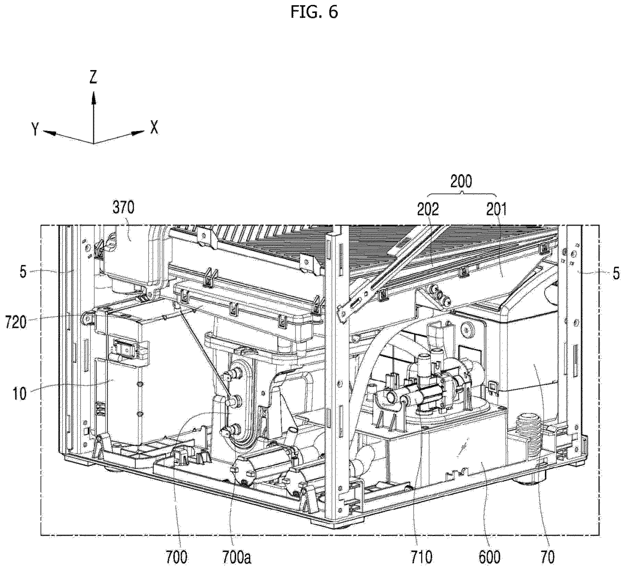

FIG. 6 is a perspective view illustrating a machine room part of the shoes care device 1 illustrated in FIG. 5.

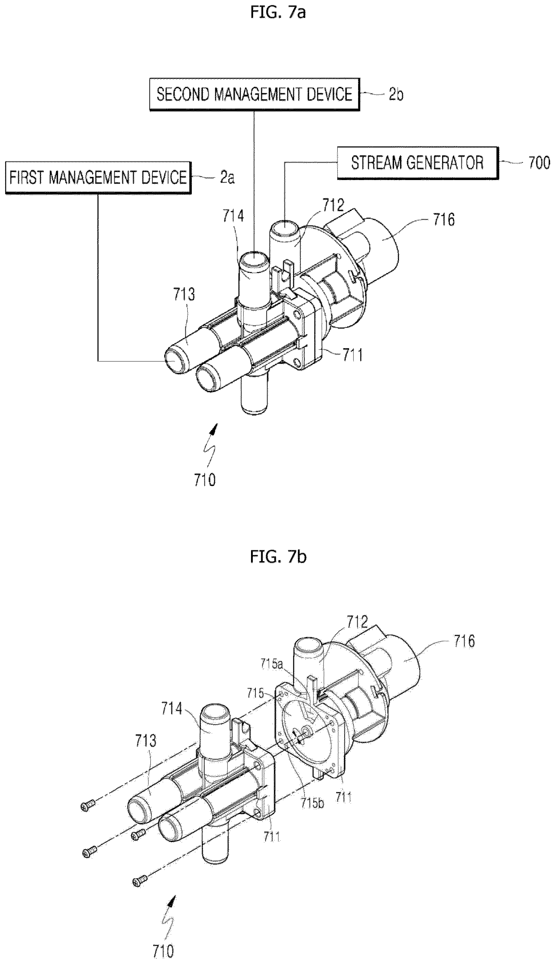

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

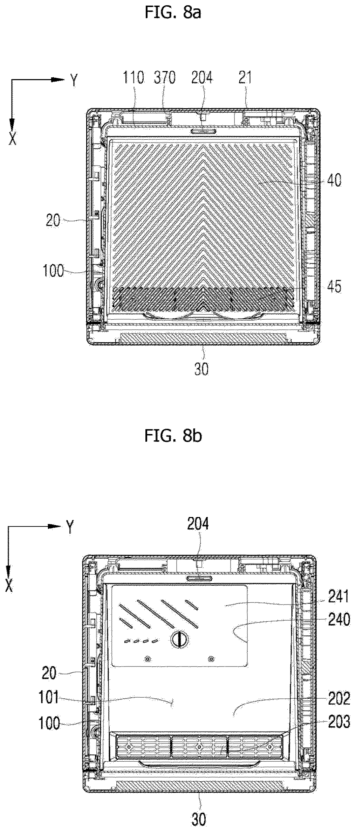

FIG. 8a is a cross-sectional view taken along line D-D' of the shoes care device 1 illustrated in FIG. 3.

FIG. 8b is a view illustrating a state in which the main shelf 40 is removed from the shoes care device 1 illustrated in FIG. 8a.

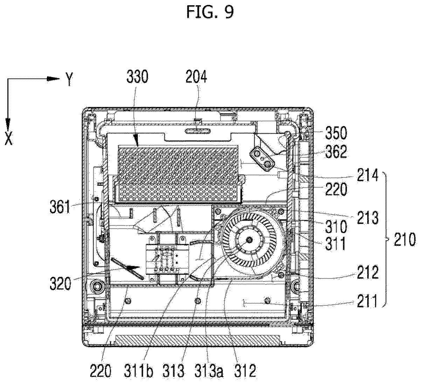

FIG. 9 is a cross-sectional view taken along line E-E' of the shoes care device illustrated in FIG. 3.

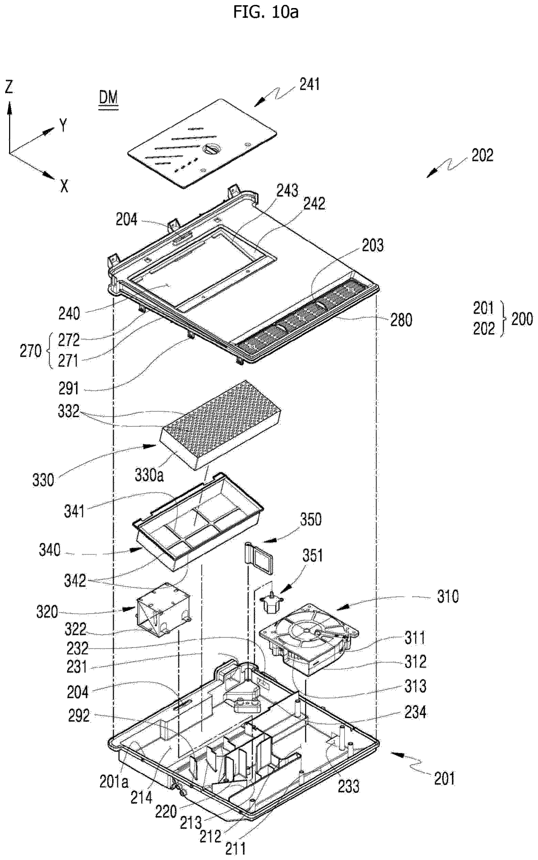

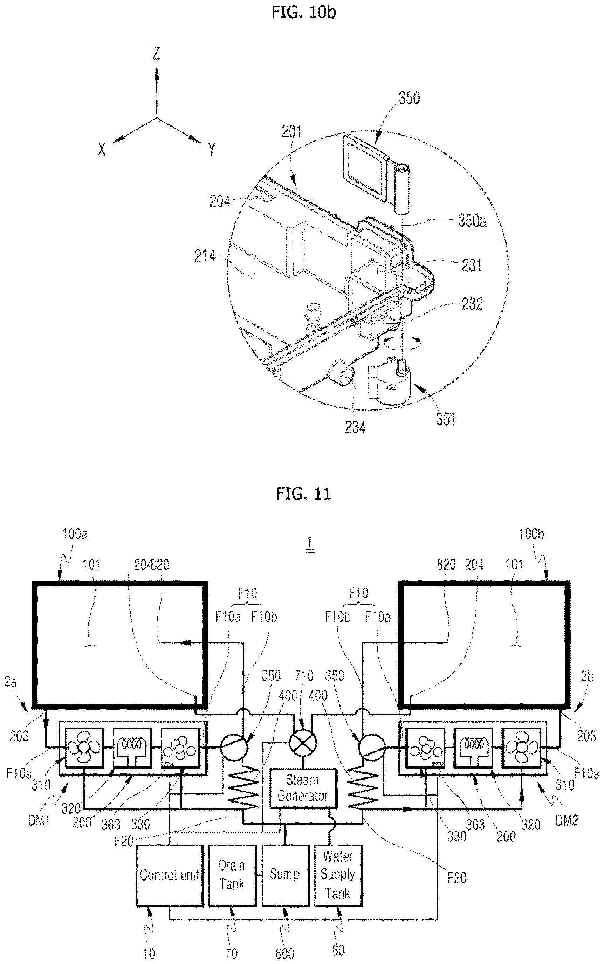

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (A) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of p the ventilation art 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device 1 according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam generator 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device 1 according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage FlOb may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoes care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

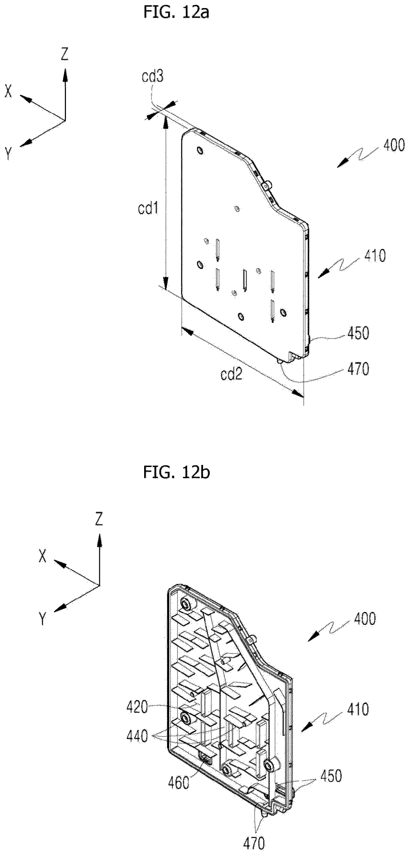

FIG. 12a is an exploded perspective view illustrating the condenser 400 according to one embodiment of the present invention.

FIG. 12B is a view illustrating an inner state of the condenser 400 of FIG. 12a.

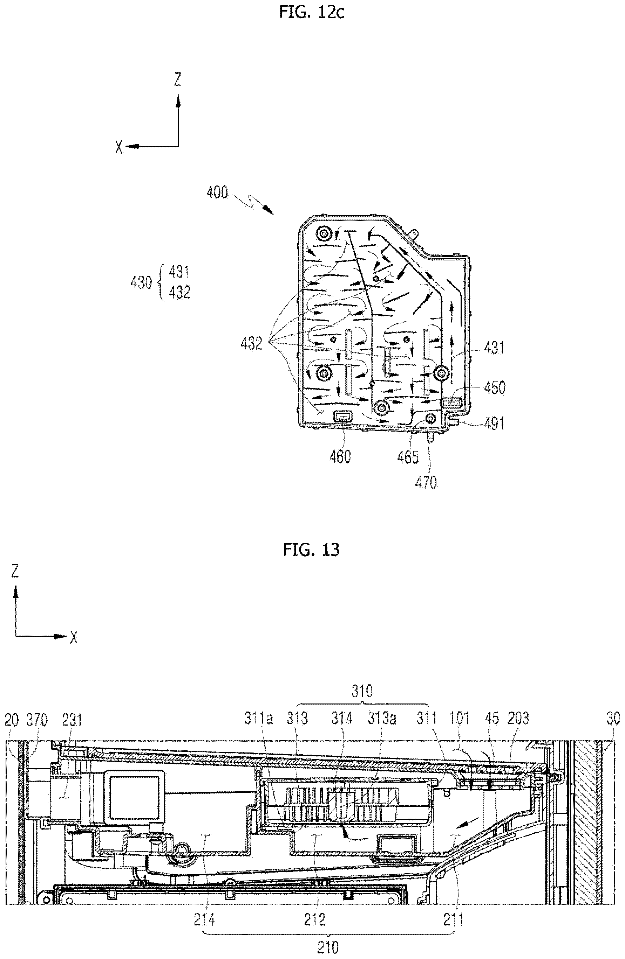

FIG. 12C is a front view illustrating the condenser 400 of FIG. 12b.

The condenser 400 forms a part of the regeneration path F20.

The condenser 400 may be made of or include a material such as synthetic resin, metal, ceramic, etc. The condenser 400 may be configured by injection molding, press molding, etc.

The condenser 400 may be made of a material having excellent thermal conductivity. The condenser 400 may be made of a metal with excellent thermal conductivity, and can be made of a material such as aluminum, an aluminum alloy, copper, a copper alloy, etc.

The condenser 400 may include a condenser housing 410, a flow path guide wall 440, a condenser inlet 450, and a condensed water outlet 470. The condenser 400 may include a condenser communication port 465.

In one embodiment of the present invention, the condenser 400 is disposed between the inner cabinet 100 and the outer cabinet 20. The condenser 400 may be disposed between the inner rear plate 110 and the outer rear plate 21, between the first inner side plate 120 and the first outer side plate 22, or between the second inner side plate 130 and the second outer side plate 23.

In the drawing in accordance with one embodiment of the present invention, the condenser 400 is illustrated to be disposed between the first inner side plate 120 and the first outer side plate 22.

A thickness cd3 of the condenser 400 (the size of the condenser 400 in the second direction (Y direction)) may be 0.5 to 1 times a distance between the first inner side plate 120 and the first outer side plate 22. A width cd2 of the condenser 400 (the size of the condenser 400 in the first direction (X direction)) and a height cd1 in the vertical direction may be 10 times or more and 40 times or less of the thickness cd3 of the condenser 400 (the size of the condenser 400 in the second direction (Y direction)), respectively.

The condenser 400 may be in close contact with the first inner side plate 120 and/or the first outer side plate 22. That is, the condenser 400 may be in close contact with an outer surface of the first inner side plate 120 or in close contact with an inner surface of the first outer side plate 22.

The first inner side plate 120 and/or the first outer side plate 22 may be made of a metal having excellent thermal conductivity. When the condenser 400 is closely coupled to the first inner side plate 120, the first inner side plate 120 may be made of a metallic material having excellent thermal conductivity, and when the condenser 400 is closely coupled to the first outer side plate 22, the first outer side plate 22 may be made of a metallic material having excellent thermal conductivity.

Accordingly, the water vapor moving through the condenser 400 may be effectively condensed.

In the shoes care device 1 according to one embodiment of the present invention, the machine room 50 is provided below the inner cabinet 100, and the condenser 400 is disposed between the inner cabinet 100 and the outer cabinet 20. That is, the condenser 400 is not provided inside the machine room 50 and is disposed outside a restricted space of the machine room 50.

In addition, the condenser 400 is disposed between the inner cabinet 100 and the outer cabinet 20 and can fully utilize the space between the inner cabinet 100 and the outer cabinet 20, and may be configured to have a large area on the whole.

In the shoes care device 1 according to one embodiment of the present invention, the condenser 400 may be disposed between the first inner side plate 120 of the inner cabinet 100 and the first outer side plate 22 of the outer cabinet 20, and the dry air duct 370 may be disposed between the inner rear plate 110 of the inner cabinet 100 and the outer rear plate 21 of the outer cabinet 20.

The condenser 400 may be formed to have a size corresponding to an area of one wall surface of the inner cabinet 100 (e.g., an area of the first inner side plate 120), and accordingly, the condenser 400 may be configured to have a relatively large area.

In one embodiment, the vertical height cd1 of the condenser 400 may be 0.5 to 1 times the vertical height h11 or h12 of the inner cabinet 100, and the width cd2 of the condenser 400 may be 0.5 to 1 times the width (the size in the first direction (X direction)) in the first inner side plate 120 of the inner cabinet 100.

According to one embodiment of the present invention, the vertical height of the machine room 50 in the shoes care device 1 may be formed relatively low, and the vertical heights h2 of the machine room 50 may be formed smaller than the vertical height h11 or h12 of the inner cabinet 100.

In this case, since the space inside the machine room 50 becomes relatively small, and the machine room 50 accommodates several components that constitute the shoes care device 1, the arrangement and design of each component may be restricted. Furthermore, unlike the present invention, when the condenser 400 is disposed inside the machine room 50, the shape and position of the condenser 400 may be limited, and the heat exchange efficiency of the condenser 400 may be reduced.

In one embodiment of the present invention, since the condenser 400 is disposed between the inner cabinet 100 and the outer cabinet 20, each component can be effectively arranged even when the vertical height of the machine room 50 needs to be formed relatively low.

In addition, by placing the condenser 400 between the inner cabinet 100 and the outer cabinet 20, the condenser 400 can be formed in a relatively thin and wide shape, the heat exchange area of the condenser 400 can be expanded, and the heat exchange between an external air of the shoes care device 1 and the condenser 400 can be easily performed. Accordingly, water vapor passing through the condenser 400 may be effectively condensed.

While the condenser 400 is disposed between the first inner side plate 120 and the first outer side plate 22, the condenser 400 may be disposed to extend upwards from the module housing 200. Further, the condenser 400 is disposed higher than the sump 600 disposed inside the machine room 50.

Accordingly, steam in the third module chamber 214 of the module housing 200 naturally rises and flows into the condenser 400, and the condensed water in the condenser 400 naturally descends by gravity and flows into the sump 600, and when the dehumidifying material 331 is regenerated, the steam can be effectively condensed and discharged.

The condenser housing 410 forms an overall appearance of the condenser 400. A condensation space 420, which is a space inside the condenser 400, is provided inside the condenser housing 410.

The condenser inlet 450 forms an inlet of the condenser 400 through which air flows into the condenser 400, and forms an inlet of the condensation space 420.

The condenser inlet 450 is connected to and in communication with the wet air outlet 232. In the shoes care device 1 according to one embodiment of the present invention, the wet air outlet 232 and the condenser inlet 450 may be directly connected to each other while being disposed inside and outside based on the first inner side plate 120 of the inner cabinet 100.

While wet air introduced into the condenser 400 through the condenser inlet 450 moves along the flow path inside the condenser 400, the wet air is condensed by heat exchange and moves below the condenser 400.

The condensed water outlet 470 forms an outlet through which the condensed water in the condenser 400 is discharged, and also forms an outlet of the condensation space 420.

The condensed water outlet 470 of the condenser 400 is connected to the sump 600, and the condensed water inside the condenser 400 moves to the sump 600.

The condensed water outlet 470 may be formed in the lower end of the condenser 400. That is, the condensed water outlet 470 may be formed in the lowest part of the condensation space 420. The lower end of the condensation space 420 may be inclined downwardly toward the condensed water outlet 470.

The condensed water outlet 470 is connected to the sump 600. The condensed water outlet 470 for the movement of the condensed water and the sump 600 may be connected by a pipe, a hose, etc.

The flow path guide wall 440 is formed in the shape of a plate having a narrow width and a long length. The flow path guide wall 440 may be provided in the inside (the condensation space 420) of the condenser housing 410 and may be formed in a plural form.

A plurality of flow path guide walls 440 are disposed to be spaced apart from each other to form a path through which water vapor and condensed water move. The plurality of flow path guide walls 440 may be disposed to cross each other.

The flow path guide wall 440 forms a condenser flow path 430 which is a path extending from the condenser inlet 450 to a condenser outlet 460 in the condensation space 420. The condenser flow path 430 is a movement passage of air (or water) provided inside the condenser 400, and connects the condenser inlet 450 and the condenser outlet 460. In addition, the condenser flow path 430 is a movement passage of air (or water) provided inside the condenser 400, and connects the condenser inlet 450 and the condensed water outlet 470.

The condenser flow path 430 may include an introduction section 431 and a condensation section 432.

As the plurality of flow path guide walls 440 are spaced apart from each other, the condensation section 432 may be provided between the flow path guide walls 440.

The condensation section 432 may be formed in a zigzag shape extending from the uppermost side of the condensation space 420 to the lower side.

In one embodiment of the present invention, an angle formed by the flow path guide wall 440 forming the condensation section 432 with a horizontal plane may be 1 to 10°. The flow path guide wall 440 constituting the condensation section 432 is formed substantially along the first direction (X direction), and may be disposed to be inclined upwards or downwards along the first direction (X direction).

In one embodiment of the present invention, the length of the flow path guide wall 440 constituting the condensation section 432 may be 50 to 400 mm, the width of the flow path guide wall 440 may be 5 to 20 mm, and an interval between the flow path guide walls 440 may be 10 to 50 mm.

In the arrangement explained above, the contact area and contact time between the steam and the condenser 400 can be increased while the water vapor moves along the zigzag-shaped condensation section 432 in the condensation space 420, and the heat exchange between the water vapor and condenser 400 and the condensation of the water vapor can be effectively performed.

In addition, by inclinedly forming the flow path guide wall 440, the condensed water formed in the condensation space 420 can move downwards along the surface of the flow path guide wall 440, and can move smoothly in the direction of gravity without accumulating inside the condenser 400, and residual water can be effectively prevented from occurring inside the condenser 400.

The introduction section 431 may extend upwards from the condenser inlet 450 to an upper start part of the condensation section 432.

The condenser outlet 460 forms an outlet of the condenser 400 through which air inside the condenser 400 is discharged.

The condenser outlet 460 may be formed in the lower part of the condenser 400. The condenser outlet 460 may be formed in a lower side of the condensation section 432.

However, the condenser outlet 460 may be formed in an area higher than the condensed water outlet 470. Accordingly, the condensed water inside the condenser 400 may be discharged through the condensed water outlet 470, and the air inside the condenser 400 may be discharged through the condenser outlet 460.

Meanwhile, in the shoes care device 1 according to one embodiment of the present invention, the inside of the condenser 400 and the inside of the module housing 200 may be configured to communicate with each other through the condenser outlet 460.

In the shoes care device 1 according to one embodiment of the present invention, the condenser outlet 460 may be formed in a position and a height corresponding to the first condensed water discharge hole 233, and the condenser outlet 460 and the first condensed water discharge hole 233 may be coupled to each other.

In the air introduced into the condenser 400, the condensed water moves to the sump 600 through the condensed water outlet 470, and uncondensed air may flow back into the module housing 200 through the condenser outlet 460.

High-temperature and humid air inside the module housing 200 forming the conversion flow path F10a flows into the condenser 400 of the regeneration path F20, the condensed water inside the condenser 400 moves to the sump 600, the uncondensed air can be resupplied into the module housing 200 forming the conversion flow path F10a, and the air is condensed while being circulated in the module housing 200 and the condenser 400.

The condenser communication port 465 communicates with the third module chamber 214 of the module housing 200 at a lower side of the condensation space. That is, the inside of the condenser 400 and the inside of the module housing 200 may be configured to communicate with each other through the condenser communication port 465.

The condenser communication port 465 may be formed directly above the condensed water outlet 470.

In the shoes care device 1 according to one embodiment of the present invention, the condenser communication hole 465 may be formed in a position and a height corresponding to the second condensed water discharge hole 234, and the condenser communication hole 465 and the second condensed water discharge hole 234 may be coupled to each other.

The condensed water inside the third module chamber 214 flows into the condenser 400 through the second condensed water discharge hole 234 and the condenser communication port 465, and moves to the sump 600 through the condensed water outlet 470.

The shoes care device 1 according to one embodiment of the present invention may include a condenser connection portion 490.

The condenser connection portion 490 may form a flow path directly connecting the condenser 400 of the first management device 2a and the condenser 400 of the second management device 2b.

The condenser connection portion 490 may be formed in the form of a pipe, a hose, etc. In the shoes care device 1 according to one embodiment of the present invention, when the condenser 400 of the first management device 2a is disposed on the upper side of the condenser 400 of the second management device 2b and the sump 600 is disposed on the lower side of the condenser 400 of the second management device 2a, the condensed water inside the condenser 400 of the first management device 2a may move into the condenser 400 of the second management device 2b through the condenser connection portion 490 and then move the sump 600.

For connection with the condenser connection portion 490, a condenser connector 491 may be formed in the condenser 400. The condenser connector 491 forms an inlet through which condensed water flows into the condenser 400.

The condenser connector 491 may be formed adjacent to the condensation space 420 of the condenser 400. The condenser connector 491 may be formed adjacent to the condensed water outlet 470 of the condenser 400.

When the condenser 400 of the first management device 2a is disposed above the condenser 400 of the second management device 2b and the sump 600 is disposed below the condenser 400 of the second management device 2b, the condenser connection portion 490 may be configured to connect the condensed water outlet 470 of the condenser 400 of the first management device 2a and the condenser connector 491 of the condenser 400 of the second management device 2b. In this case, the condenser connector 491 is not formed in the condenser 400 of the first management device 2a, or the condenser connector 491 of the condenser 400 of the first management device 2a may be blocked with a separate stopper.

The condensed water inside the condenser 400 of the first management device 2a may move into the condenser 400 of the second management device 2b through the condenser connection portion 490 and then move to the sump 600.

Accordingly, when the first management device 2a and the second management device 2b are arranged vertically in the shoes care device 1, a flow path (such as a hose) connecting the condenser 400 of the second management device 2b and the sump 600 may be used as a flow path configured to discharge the condensed water of the first management device 2a. Hence, the length of the flow path (hose, etc.) required for discharging the condensed water can be formed short on the whole, and only the condenser 400 of the second management device 2b may be directly connected to the sump 600 to discharge the condensed water of all the condensers 400. As a result, since an overall coupling structure can be simplified, which makes it easy to assemble and maintain the shoes care device 1, and the condensed water may be easily discharged.

The sump 600 is configured to have a structure of a container capable of accommodating and storing water. The sump 600 is configured to accommodate the condensed water generated in the shoes care device 1 therein. The sump 600 is configured to accommodate the condensed water condensed by the condenser 400.

The sump 600 may be positioned below the dehumidifying agent. The sump 600 may be positioned below the module housing 200.

The sump 600 may be positioned in the machine room 50, and formed at a lowermost portion of the shoes care device 1.

The air introduced into the inside of the module housing 200 from the inner cabinet 100 may contain relatively much moisture in a process of refreshing the shoes, and some of the air introduced into the module chamber 210 of the module housing 200 may be condensed inside the module housing 200. Further, the condensed water inside the inner cabinet 100 may be suctioned into the inside of the module housing 200 jointly in addition to the air. In this case, the condensed water may be introduced into the inside of the condenser 400 through the first condensed water discharge hole 233 and/or the second condensed water discharge hole 234 while flowing along the bottom surface of the module housing 200, and thereafter, moved and collected to the sump 600 through the condensed water outlet 470, and then discharged to the drain tank 70 or discharged to the outside or pressure-fed to the steam generator 700.

As such, the shoes care device 1 according to the embodiment may form a shoes care device 1 in which the condensed water is easily managed and discharged.

The inner cabinet 100 may be configured in various shapes and structures within a range in which the shoes are positioned in the accommodation space 101.

The machine room 50 may be positioned below the inner cabinet 100 (inside the cabinet).

Steam which is supplied to the accommodation space 101 of the inner cabinet 100 has a property to ascend. In the shoes care device 1 according to the embodiment of the present invention, the machine room 50 I positioned below the inner cabinet 100, and as a result, the steam may naturally move to the inner cabinet 100 from the machine room 50, i.e., upward, and the steam may be smoothly supplied.

In the shoes care device 1 according to the embodiment of the present invention, an effective shoes care device may be configured in which zeolite effective for dehumidification, deodorization, and humidification is placed inside the module housing 200, air passing through the zeolite is circulated in a connection path F10 while being re-introduced into the inside of the inner cabinet 100, and further, the condensed water is discharged through a regeneration path F20 at the time of regenerating the zeolite.

FIG. 13 is a cross-sectional view illustrating a first module chamber of a module housing in the shoes care device 1 according to one embodiment of the present invention.

The blowing part 310 forms a part of the connection path F10. The blowing part 310 is configured to generate an air flow in the connection path F10.

The blowing part 310 may include the blowing fan 313, a blowing housing 311, and a blowing motor 314.

The blowing fan 313 may be configured to rotate around a rotation axis 313a in the third direction (Z direction) perpendicular to the first direction (X direction). The blowing motor 314 of the blowing part 310 rotates the blowing fan 313.

The blowing housing 311 is configured to accommodate the blowing fan 313. The blowing housing 311 may have a substantially round shape around the rotation axis 313a of the blowing fan 313. The blowing housing 311 may be formed in a circular shape or a spiral shape around the rotation axis 313a of the blowing fan 313.

An inlet 311a of the blowing part 310 is formed on a bottom surface of the blowing housing 311, and air flows into the blowing housing 311 below the bottom surface of the blowing housing 311.

The size of the blowing housing 311 in the horizontal direction may be formed larger than the size of the blowing housing 311 in the vertical direction. The size of the blowing housing 311 in the horizontal direction may be made more than twice the size of the blowing housing 311 in the vertical direction.

In the shoes care device 1 according to one embodiment of the present invention, when the blowing fan 313 rotates around a vertical rotation axis to allow the module housing 200 to have a relatively low vertical height, the diameter of the blowing housing 311 and the diameter of the blowing fan 313 can be formed sufficiently large, and the flow rate of air transported by the blowing part 310 can be sufficiently increased.

The blowing housing 311 may communicate with the first module chamber 212 on the rotating axis 313a of the blowing fan 313, and the edge thereof may be connected to and communicate with the blowing duct 312. The blowing duct 312 forms an outlet 311b of the blowing part 310.

Accordingly, the air below the blowing housing 311 in the first module chamber 212 moves upwards near the rotation axis 313a of the blowing fan 313 and flows into the blowing housing 311, and the air inside the blowing housing 311 is pressurized to the edge of the blowing housing 311 depending on the rotation of the blowing fan 313 and moves along the circumferential direction of the blowing housing 311 toward the blowing duct 312.

The blowing housing 311 includes the blowing duct 312 forming the outlet 311b of the blowing housing 311. The blowing duct 312 may extend in a horizontal direction from the blowing housing 311. The blowing duct 312 may be formed substantially along the second direction (Y direction). The blowing duct 312 may extend to the second module chamber 213, and all air discharged from the blowing part 310 through the blowing duct 312 may move to the second module chamber 213.

The blowing housing 311 and the blowing duct 312 may form a spiral passage around the rotating axis 313a of the blowing fan 313 together such that, when the blowing fan 313 rotates, the air inside the blowing housing 311 naturally moves to the blowing duct 312.

Along the rotation direction of the blowing fan 313, the distance in the radial direction from the rotation axis 313a of the blowing fan 313 may be sequentially increased from the blowing housing 311 to the blowing duct 312. In one embodiment, as illustrated in FIG. 9, the distance from the rotation axis 313a of the blowing fan 313 to the outer edges of the blowing housing 311 and the blowing duct 312 may be configured to increase sequentially in the clock direction.

In addition, the second module chamber 213 may be configured to extend from an end of the blowing duct 312.

In addition, the direction of sequentially connecting the first module chamber 212, the second module chamber 213, the third module chamber 214 and the dry air outlet 231 may be configured to correspond to the direction of the movement of air around the rotation axis of the blowing fan 313 in the blowing housing 311.

Based on FIG. 9, the rotation direction of the blowing fan 313 may be configured to be clockwise, the distance from the rotation axis 313a of the blowing fan 313 to the outer edges of the blowing housing 311 and the blowing duct 312 may be configured to sequentially increase in the clockwise direction and have a spiral form, and the direction of sequentially connecting the first module chamber 212, the second module chamber 213, the third module chamber 214 and the dry air outlet 231 may be configured to be clockwise.

Accordingly, the air passing through the blowing housing 311 and the blowing fan 313 may move in the clockwise direction inside the module housing 200 toward the dry air outlet 231 or the wet air outlet 232, and the air may move naturally along the formation direction of the flow path (the convention flow path F10a) inside the module housing 200.

In addition, by performing the blowing part 310 and the blowing duct 312 as described above, the flow rate of the air supplied to the third module chamber 214 can be stably secured, and sufficient air can be supplied to the dehumidifying part 330.

In addition, while the air moving through the third module chamber 214 smoothly passes through the dehumidifying part 330, a flow path resistance of the air passing through the dehumidifying part 330 can be prevented from unnecessarily increasing.

In the shoes care device 1 according to one embodiment of the present invention, the blowing part 310 may be spaced apart from the bottom of the first module chamber 212 such that air in the first module chamber 212 flows into the blowing part 310 from the lower side of the first module chamber 212. Accordingly, even if condensed water is generated inside the first module chamber 212, the condensed water can move along the bottom surface of the first module chamber 212 and be discharged to the outside of the module housing 200, and the residual water can be prevented or minimized in the blowing part 310.

In the shoes care device 1 according to one embodiment of the present invention, the direction of sequentially connecting the first module chamber 212, the second module chamber 213, the third module chamber 214 and the dry air outlet 231 may correspond to the direction of movement of air around the rotation axis of the blowing fan 313 in the blowing housing 311. Accordingly, the air introduced into the blowing housing 311 can naturally move into the module housing 200 in a predetermined direction from the blowing housing 311 to the second module chamber 213, the third module chamber 214 and the dry air outlet 231 with respect to the rotation axis of the blowing fan 313. Accordingly, a smooth air flow may be achieved in the module housing 200, and an unintentional increase in flow resistance can be prevented.

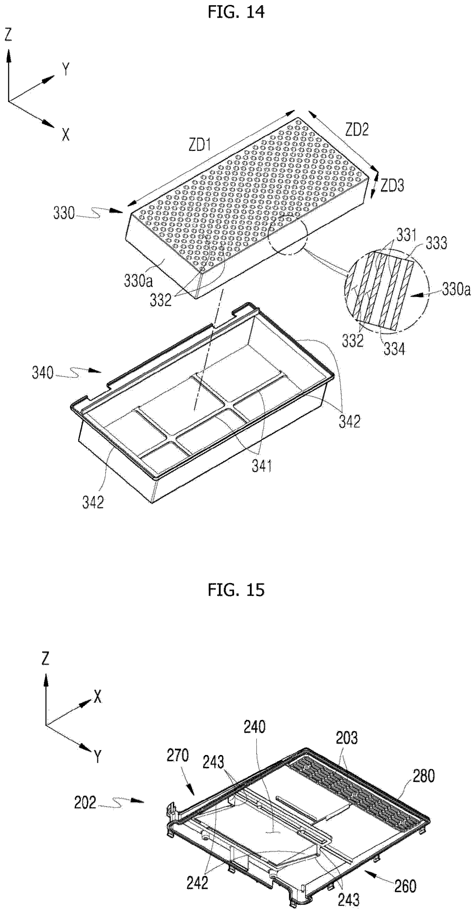

FIG. 14 is a perspective view illustrating a dehumidifying part and a dehumidifying material housing according to one embodiment of the present invention.

FIG. 15 is a bottom perspective view illustrating a module cover according to one embodiment of the present invention.

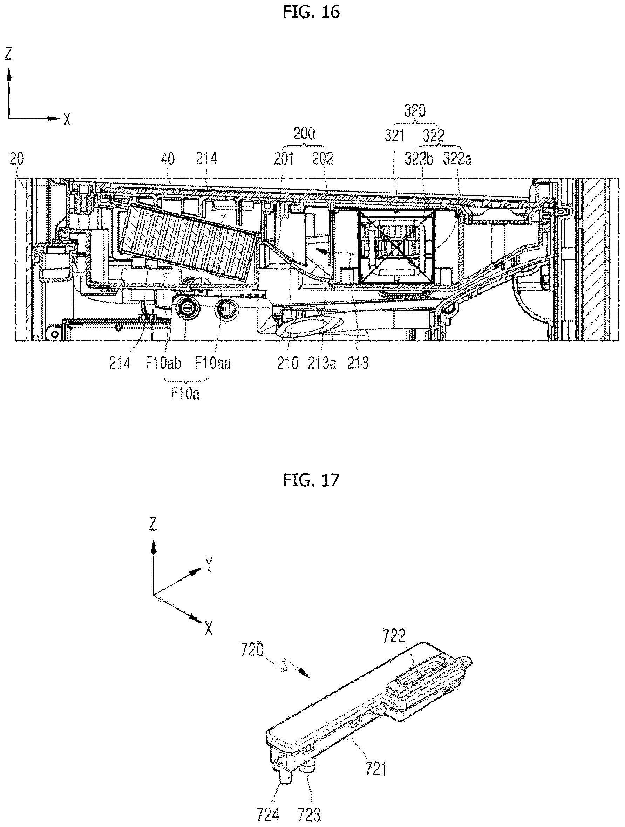

FIG. 16 is a cross-sectional view illustrating a part of a third module chamber of the module housing in the shoes care device 1 according to the present invention.

The dehumidifying part 330 according to one embodiment of the present invention may be configured to have a predetermined thickness and length. In one embodiment, as described above, the dehumidifying part 330 may be configured to have a substantially hexahedral shape.

Accordingly, the dehumidifying part 330 may have a predetermined length, width, and thickness.

Each of a length ZD1 and a width ZD2 of the dehumidifying part 330 may be formed longer than a thickness ZD3 of the dehumidifying part 330. In one embodiment, the length ZD1 and the width ZD2 of the dehumidifying part 330 be made more than twice the thickness ZD3 of the dehumidifying part 330. In addition, the length ZD1 of the dehumidifying part 330 may be formed longer than the width ZD2 of the dehumidifying part 330.

The dehumidifying part 330 includes an upper surface 333 and a lower surface 334 facing each other. Here, the upper surface 333 of the dehumidifying part 330 and the lower surface 334 of the dehumidifying part 330 are opposite surfaces facing each other in the thickness direction of the dehumidifying part 330.

As described above, the dehumidifying part 330 is provided with the plurality of dehumidification through holes 332. The dehumidifying through hole 332 may be configured to penetrate the dehumidifying part 330 in a direction in which the upper surface 333 and the lower surface 334 of the dehumidifying part 330 are connected to each other.

In the shoes care device 1 according to one embodiment of the present invention, the dehumidifying part 330 may be accommodated inside the module housing 200 while being accommodated in the dehumidifying material housing 340.

The dehumidifying material housing 340 is formed in the form of a container capable of accommodating the dehumidifying part 330. In a state where the dehumidifying part 330 is accommodated in the dehumidifying material housing 340, an edge wall of the dehumidifying material housing 340 may be in close contact with the dehumidifying part 330. Accordingly, the dehumidifying part 330 can be prevented from being separated inside the dehumidifying material housing 340.

The dehumidifying material housing 340 has an upper side opened and a lower side opened. However, the lower side of the dehumidifying material housing 340 is provided with a support 341 that supports the dehumidifying part 330 such that the dehumidifying part 330 does not deviate downwards. The supports 341 may be disposed to cross each other in the form of a grid or a net. The gap (opening) between the supports 341 is formed sufficiently larger than the dehumidifying through hole 332 so as not to interfere with the flow of the air passing through the dehumidifying part 330.

In the shoes care device 1 according to one embodiment of the present invention, when the dehumidifying part 330 is disposed inside the module housing 200, the dehumidifying part 330 may be disposed so that the upper surface 333 and the lower surface 334 are inclined without being parallel to the horizontal direction.

The dehumidifying part 330 may be disposed in the third module chamber 214 in a form inclined downwardly toward the second module chamber 213. That is, the upper surface 333 and the lower surface 334 of the dehumidifying part 330 may be disposed to be inclined downwardly toward the second module chamber 213.

Since the dehumidifying part 330 is inclined downwardly toward the second module chamber 213 in the third module chamber 214, the air before penetrating the dehumidifying part 330 is disposed in an upper space of the dehumidifying part 330 in the third module chamber 214, and the air after penetrating the dehumidifying part 330 is disposed in a lower space of the dehumidifying part 330 in the third module chamber 214. Here, the upper space of the dehumidifying part 330 in the third module chamber 214 is defined as 'a first flow path F10aa', and the lower space of the dehumidifying part 330 in the third module chamber 214 is defined as 'a second flow path F10ab.

In addition, since the dehumidifying part 330 is disposed to be inclined in the third module chamber 214, the dehumidifying through hole 332 is also disposed to be inclined.

A cover partition wall 242 may be formed in the module cover 202.

The cover partition wall 242 is formed in a plate shape extending downwards from the edge of the dehumidifying material exit 240. The cover partition wall 242 may be formed in a substantially triangular plate shape.

The cover partition wall 242 may be configured such that a lower edge thereof is inclined downwards from the third module chamber 214 to the second module chamber 213. A pair of cover partition walls 242 may be provided to be spaced apart from each other in the left-right direction. The distance between the pair of cover partition walls 242 may be configured to correspond to the length of the dehumidifying part 330.

A lower locking portion 243 may be formed in a lower edge of the cover partition wall 242 and a front edge of the cover partition wall 242 in the first direction (X direction), and an upper locking portion 342 may be formed in an upper edge of the dehumidifying material housing 340 to get settled and locked in an upper side of the lower locking portion 243. Accordingly, when the dehumidifying material housing 340 is settled in the cover partition wall 242 in a state where the dehumidifying part 330 is accommodated in the dehumidifying material housing 340, the upper locking portion 342 of the dehumidifying material housing 340 is settled and assembled in an upper side of the lower locking portion 243 of the cover partition wall 242.

In addition, in this case, the cover partition wall 242 may block direct communication between the first flow path F10aa and the second flow path F10ab while the lower edge thereof is in close contact with the upper edge of the dehumidifying part 330.

By providing the cover partition wall 242 in the module cover 202, the air in the first flow path F10aa may pass through the dehumidifying part 330 over the entire area of the dehumidifying part 330 and move to the second flow path F10ab. In addition, a plurality of dehumidifying through holes 332 penetrating the dehumidifying part 330 in the thickness direction may be formed in the dehumidifying part 330, thereby increasing the contact area between the air passing through the third module chamber 214 and the dehumidifying material 331.

In one embodiment of the present invention, the dehumidifying part 330 is not disposed parallel to the horizontal direction and is inclined downwardly toward the second module chamber 213. Comparing this with the case where the dehumidifying part 330 is disposed horizontally, the size of the upper surface of the dehumidifying part 330 may be formed larger, and the entire volume of the dehumidifying part 330 may be increased. Accordingly, the amount of dehumidification of air by the dehumidifying part 330 may be increased.

As described above, as the dehumidifying part 330 is disposed to be inclined, the direction from the first flow path F10aa to the second flow path F10ab through the dehumidifying part 330 is configured not to be vertical but to be inclined. A rapid change in the direction of air in a path through which the air moves to the second module chamber 213, the first flow path F10aa, and the second flow path F10ab can be reduced to achieve a smooth movement of air.

Accordingly, the natural movement of air moving through the dehumidifying part 330 can be achieved, and an unnecessary flow path resistance can be minimized in the third module chamber 214.

In addition, while the air of the second module chamber 213 moves from the first flow path F10aa to the second flow path F10ab, moisture (small droplets or condensed water) may enter or occur in the third module chamber 214. The bottom surface of the dehumidifying part 330 may be naturally separated from the bottom of the third module chamber 214, and the air inside the third module chamber 214 may move downwards through the dehumidifying part 330 from the upper side of the third module chamber 214. Accordingly, the small droplets or the condensed water can move along the bottom surface of the third module chamber 214 without remaining or seeping into the dehumidifying part 330, and can be easily discharged toward the condenser 400.

In one embodiment, the dehumidifying part 330 may have a constant cross-section along the second direction (Y direction).

In another embodiment, the dehumidifying part 330 may be formed in a form where the thickness thereof is deformed along the second direction (Y direction).

In the shoes care device 1 according to one embodiment of the present invention, the first module chamber 212 and the second module chamber 213 are formed in front of the third module chamber 214 with respect to the first direction (X direction). That is, when the longitudinal direction of the dehumidifying part 330 is formed along the second direction (Y direction), the first module chamber 212 or the second module chamber 213 does not interfere with securing the length of the third module chamber 214, and the blowing part 310 or the heating part 320 does not interfere with securing the length of the dehumidifying part 330. Accordingly, a total length ZD1 of the dehumidifying part 330 may be formed longer than the length of each of the blowing part 310 and the heating part 320 with respect to the second direction (Y direction).

Accordingly, the length of the dehumidifying part 330 can be secured sufficiently long, the entire volume of the dehumidifying part 330 can be formed relatively large, and the dehumidifying amount per unit time of the dehumidifying part 330 can be improved.

In the shoes care device 1 according to one embodiment of the present invention, the length ZD1 of the dehumidifying part 330 may be longer than 1/2 of the length of each of the inner cabinet 100 and the module housing 200 with respect to the second direction (Y direction).

The dehumidifying part 330 may be spaced apart from the bottom of the third module chamber 214 so that the air inside the third module chamber 214 moves downwards through the dehumidifying part 330 from the upper side of the third module chamber 214. Since the dehumidifying part 330 is spaced apart from the bottom of the third module chamber 214, the air inside the third module chamber 214 can smoothly pass through the dehumidifying part 330, and the condensed water generated by penetrating the dehumidifying part 330 moves along the bottom surface of the third module chamber 214 and can be discharged outside the module housing 200.

In the shoes care device 1 according to one embodiment of the present invention, the dehumidifying part 330 provided in the third module chamber 214 may be disposed to be inclined. The dehumidifying part 330 may be disposed to be inclined downwardly toward the second module chamber 213. Accordingly, when the air moves from the second module chamber 213 to the third module chamber 214, the air can easily pass through the dehumidifying part 330. In addition, compared to the case where the dehumidifying part 330 is horizontally arranged, the area or volume of the dehumidifying part 330 can be further expanded, and the dehumidifying amount per unit time of the dehumidifying part 330 can be improved.

FIG. 17 is a perspective view illustrating a steam separator according to one embodiment of the present invention.

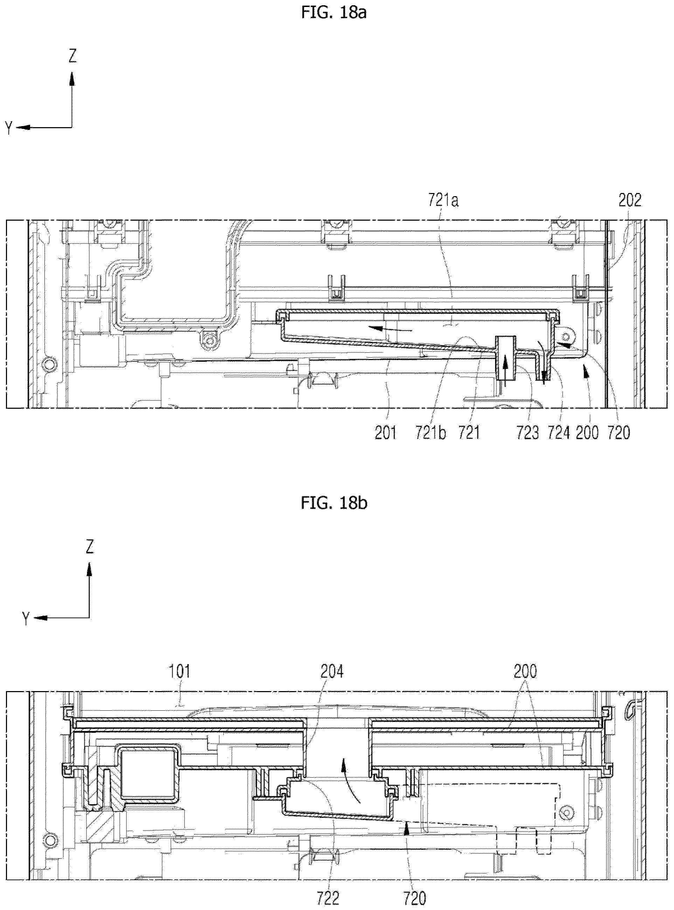

FIG. 18a is a cross-sectional view illustrating a part of a separating inlet of a steam separator in the shoes care device 1 according to one embodiment of the present invention.

FIG. 18b is a cross-sectional view illustrating a part of a separating connector of the steam separator in the shoes care device 1 according to one embodiment of the present invention.

The shoes care device 1 according to one embodiment of the present invention may further include the steam separator 720 installed on the steam inlet 204.

Most of the steam supplied from the steam generator 700 to the accommodation space 101 of the inner cabinet 100 is in a gaseous state, but the stream can be condensed during the movement to generate condensed water in a liquid state.

As described above, measures are needed to prevent moisture from leaving at an unintended part inside the shoes care device 1.

This is applicable to the flow path through which steam is supplied, and the condensed water in the steam needs to be prevented from being adsorbed and remaining on an inner surface of the flow path through which steam is supplied.

In addition, even if the condensed water in steam is supplied to the inside of the inner cabinet 100 without change, since the condensed water is not supplied to the shoes in the form of steam, it is difficult to properly perform the management on the shoes (sterilization treatment by high-temperature steam, swelling of shoe materials, etc.).

Therefore, preferably, the condensed water in the steam is removed through the steam separator 720 installed in the steam inlet 204 before the condensed water in the steam flows into the steam inlet 204.

In the shoes care device 1 according to one embodiment of the present invention, the steam separator 720 may include a separating base 721, the separating connector 722, the separating inlet 723, and a separating outlet 724.

The separating base 721 is formed in a housing shape forming a predetermined inner space 721a. The separating base 721 may have an inner area relatively larger than the hole formed by the steam inlet 204 in a plan view, and the separating base 721 may be fixed to the module housing 200 while covering the steam inlet 204 in the lower side of the steam inlet 204.

The separating inlet 723 forms an inlet through which steam flows into the steam separator 720. The separating inlet 723 may be connected to the steam valve 710, and the steam generated by the steam generator 700 may flow into the steam separator 720 through the separating inlet 723 after passing through the steam valve 710. The separating inlet 723 may be disposed lower than the separating connector 722. The separating inlet 723 may be formed in a shape opened vertically from the lower side of the separating base 721.

The separating inlet 723 may have a vertical tubular shape, and an upper end thereof may be formed higher than a bottom surface 721b of the separating base 721. The separating inlet 723 may be formed to protrude upward from the bottom surface 721b of the separating base 721. Accordingly, even if the condensed water occurs inside the separating base 721 and flows to the bottom surface 721b of the separating base 721, the condensed water may be prevented from flowing into the separating inlet 723, and all the condensed water may be discharged to the separating outlet 724, as described below.

The separating connector 722 forms an outlet through which steam inside the steam separator 720 is discharged. The steam inside the steam separator 720 may move to the steam inlet 204 through the separating connector 722. The separating connector 722 may be formed above the separating base 721. The separating connector 722 can be formed in a form opened from the upper side of the separating base 721 to the upper side in the vertical direction, and can be directly connected to and communicate with the steam inlet 204.

The separating outlet 724 forms an outlet through which condensed water inside the steam separator 720 is discharged. The separating outlet 724 is connected to the sump 600, and the condensed water inside the steam separator 720 may move to the sump 600 through the separating outlet 724. The separating outlet 724 may be disposed lower than the separating connector 722. The separating outlet 724 may be formed in a form opened from the lower side of the separating base 721 to the lower side in the vertical direction.

The separating outlet 724 may be formed in a lower end of the bottom of the separating base 721. That is, the separating outlet 724 may be formed at the lowest part among the bottom surfaces of the separating base 721. The bottom surface of the separating base 721 may be inclined downwardly toward the separating outlet 724. In one embodiment, the bottom surface of the separating base 721 may be inclined downward in the second direction (Y direction) or a direction opposite to the second direction (Y direction), and the separating outlet 724 may be formed on the bottom surface of the separating base 721 at a front end or a rear end of the second direction (Y direction).

The steam flowing into the steam separator 720 may be heated to a predetermined temperature, and may be above ordinary temperature (e.g., 20±5°C). Since this steam has a strong tendency to rise, it can move naturally through the separating connector 722 formed upwards from the upper side of the separating base 721.

Some of the steam flowing into the steam separator 720 may be cooled and condensed inside the separating base 721, and the condensed water may flow along the bottom surface of the separating base 721, and may be discharged to the outside of the steam separator 720 through the separating outlet 724 and move to the sump 600.

As described above, in the shoes care device 1 according to one embodiment of the present invention, since the steam separator 720 is provided with the separating connector 722 and the separating outlet 724 separately, when the steam and condensed water are present together, each of them can move through an individual path

In addition, the separating connector 722 is formed in the upper side of the steam separator 720, while the separating outlet 724 is formed in the lower side of the steam separator 720. Accordingly, according to the properties of the steam and the condensed water, the water vapor and condensed water are discharged in opposite directions, and residual water can be prevented from occurring in the flow path through which the steam is supplied.

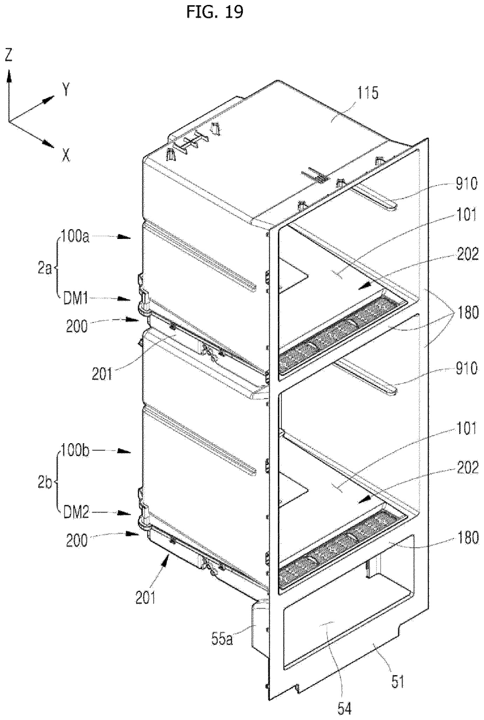

FIG. 19 is a perspective view illustrating a state in which inner cabinets 100a and 100b and the module housing 200 are coupled according to one embodiment of the present invention.

FIG. 20 is a perspective view illustrating the inner cabinets 100a and 100b illustrated in FIG. 19.

FIG. 21 is a perspective view of the inner cabinets 100a and 100b illustrated in FIG. 20 when viewed from the back.

As described above, it will be understood that the 'inner cabinet 100' described in one embodiment of the present invention means each of the 'first inner cabinet 100a' and the 'second inner cabinet 100b', except for a particularly limited case.

In the shoes care device 1 according to one embodiment of the present invention, the module housing 200 may be detachably coupled to the inner cabinet 100. The module housing 200 may be coupled to a lower side of the inner cabinet 100 while sliding in a horizontal direction. The module cover 202 is coupled to the lower end of the inner cabinet 100 so as to slide in the horizontal direction.

The module housing 200 may be coupled to a lower side of the inner cabinet 100 while moving in the first direction (X direction), and the module housing 200 may be separated from the inner cabinet 100 while moving an opposite direction to the first direction (X direction) in a state where the module housing 200 and the inner cabinet 100 are coupled to each other.

The module housing 200 may be coupled to the inner cabinet 100 while sliding in the first direction (X direction). To this end, the inner cabinet 100 may include sliding guides 160 and 170, and the module housing 200 may include sliders 260 and 270.

The sliders 260 and 270 are slidably moved along the formation direction of the sliding guides 160 and 170, and the sliders 260 and 270 and the sliding guides 160 and 170 are slidably coupled to each other.

According to one embodiment of the present invention, the module housing 200 may be formed directly under the inner cabinet 100 to prevent the connection path F10 from being unnecessarily elongated, and air in the accommodation space 101 may be rapidly dehumidified by the dehumidifying part 330. In addition, the inner cabinet 100 and the module housing 200 may be stably coupled to each other to form the shoes care device 1 with a simple structure.

In the shoes care device 1 according to one embodiment of the present invention, the blowing part 310, the heating part 320, and the dehumidifying part 330 are accommodated together in the module chamber 210 inside the module housing 200, and the module housing 200 is detachably coupled to the lower side of the inner cabinet 100. That is, the drying module DM is detachably coupled to the lower side of the inner cabinet 100. The drying module A (DM1) of the first management device 2a is detachably coupled to a lower side of the first inner cabinet 100a, and the drying module B (DM2) of the second management device 2b is detachably coupled to a lower side of the second inner cabinet 100b.

In the module housing 200, the module case 201 may be formed by injection molding, and the module case 201 may be integrally formed. The air inside the inner cabinet 100 moves to the module chamber 210 of the module housing 200, and the blowing part 310, the heating part 320, and the dehumidifying part 330, which are the main means for drying the air inside the inner cabinet 100 and the main means for regenerating the dehumidifying part 330, are disposed together in the module chamber 210 of the module housing 200. Accordingly, the condensed water generated inside the inner cabinet 100 may flow into the module chamber 210 inside the module housing 200, and the condensed water generated through the blowing part 310, the heating part 320, and/or the dehumidifying part 330 can be effective prevented from leaking from the module housing 200.

In addition, according to one embodiment of the present invention, the shoes care device 1 can be provided with a structure capable of easily managing and replacing the module housing 200 and the components accommodated therein.

Unlike one embodiment of the present invention, when the blowing part 310, the heating part 320, and the dehumidifying part 330 are accommodated in separate chambers physically separated from each other, and the chambers are coupled to each other, a potential leak at the coupling part of each chamber may not be excluded, and the coupling with the inner cabinet 100 may be cumbersome.

In the shoes care device 1 according to one embodiment of the present invention, the module housing 200 is coupled to the lower side of the inner cabinet 100 while sliding in the horizontal direction. Accordingly, when the module housing 200 is coupled to or separated from the lower side of the inner cabinet 100, there is no need to invade other spaces of the inner cabinet 100 or the machine room 50, and assembly and separation are facilitated. Further, there is no need to have a separate space for coupling or separation of the module housing 200 inside the inner cabinet 100 or inside the machine room 50. Furthermore, an increase in the vertical height of the machine room 50 can be prevented by the module housing 200.

In the shoes care device 1 according to one embodiment of the present invention, the module chamber 210 of the module housing 200 includes a first module chamber 212 accommodating the blowing part 310, a second module chamber 213 accommodating the heating part 320, and a third module chamber 214 accommodating the dehumidifying part 330. The first module chamber 212, the second module chamber 213, and the third module chamber 214 are formed at different positions in a plan view. Therefore, the shoes care device 1 can be formed which has a structure capable of minimizing the vertical height of the module housing 200 and securing the space in the inner cabinet 100 and/or the machine room 50.

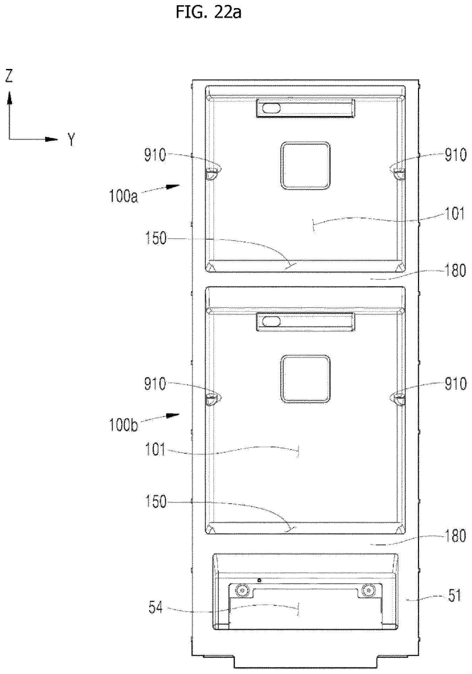

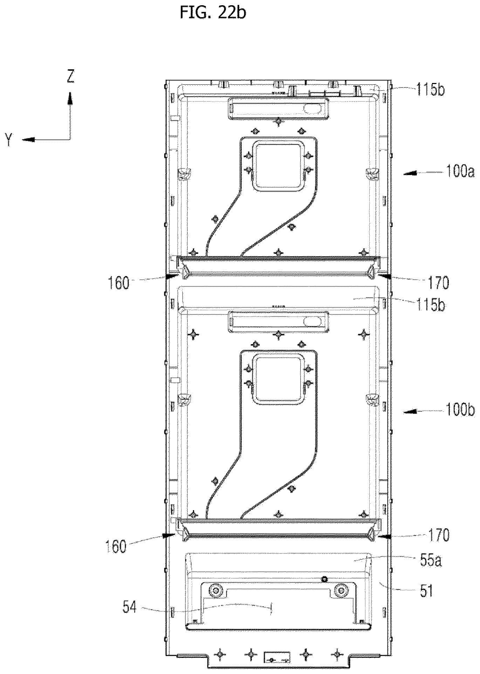

FIG. 22a is a front view illustrating the inner cabinets 100a and 100b illustrated in FIG. 20, and FIG. 22a is a rear view illustrating the inner cabinets 100a and 100b illustrated in FIG. 20.

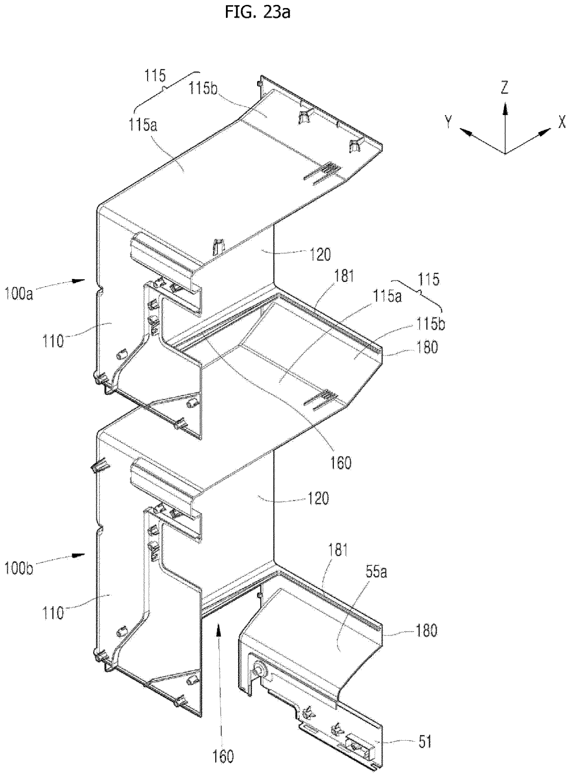

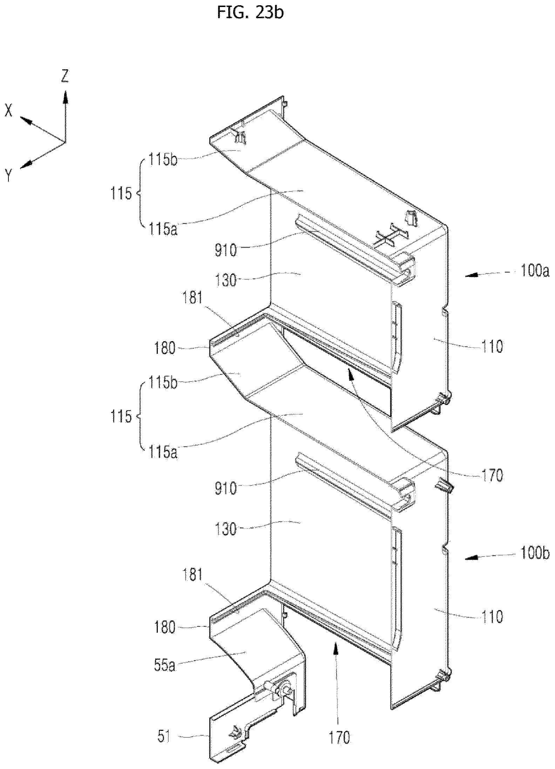

FIGS. 23a and 23b are cross-sectional perspective views illustrating the inner cabinets 100a and 100b illustrated in FIG. 20.

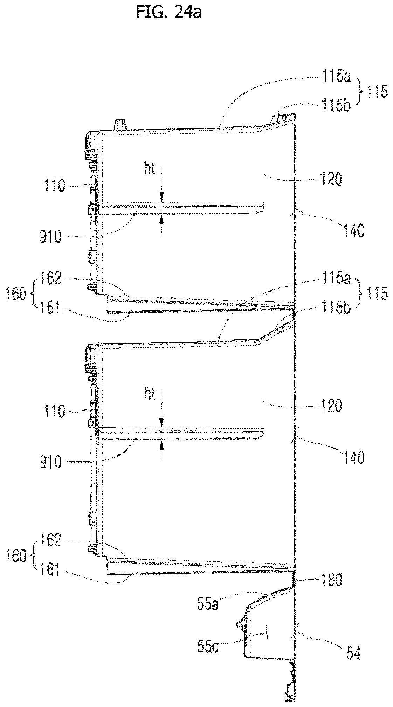

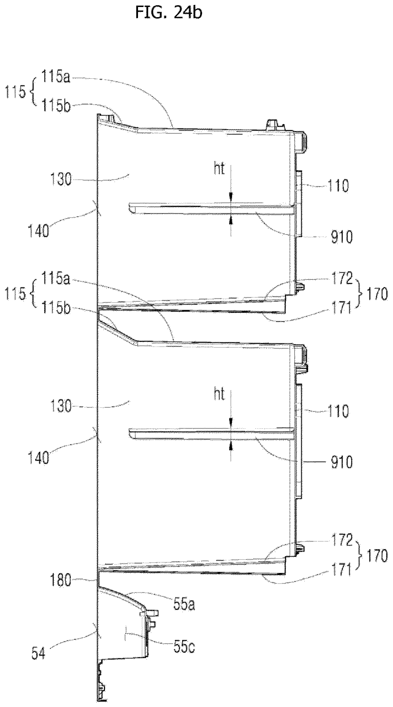

FIGS. 24a and 24b are side cross-sectional views illustrating the inner cabinets 100a and 100b illustrated in FIG. 20.

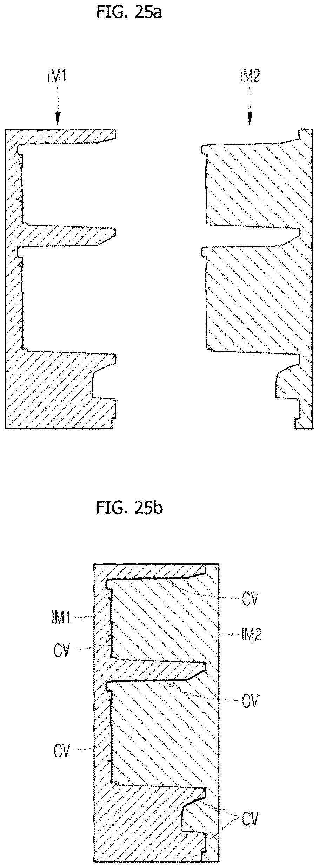

FIG. 25a is a cross-sectional view schematically illustrating a molds IM1 and IM2 used in manufacturing the inner cabinets 100a and 100b according to one embodiment of the present invention, and FIG. 25b is a schematic view illustrating a state in which the molds IM1 and IM2 of FIG. 25a is closed.

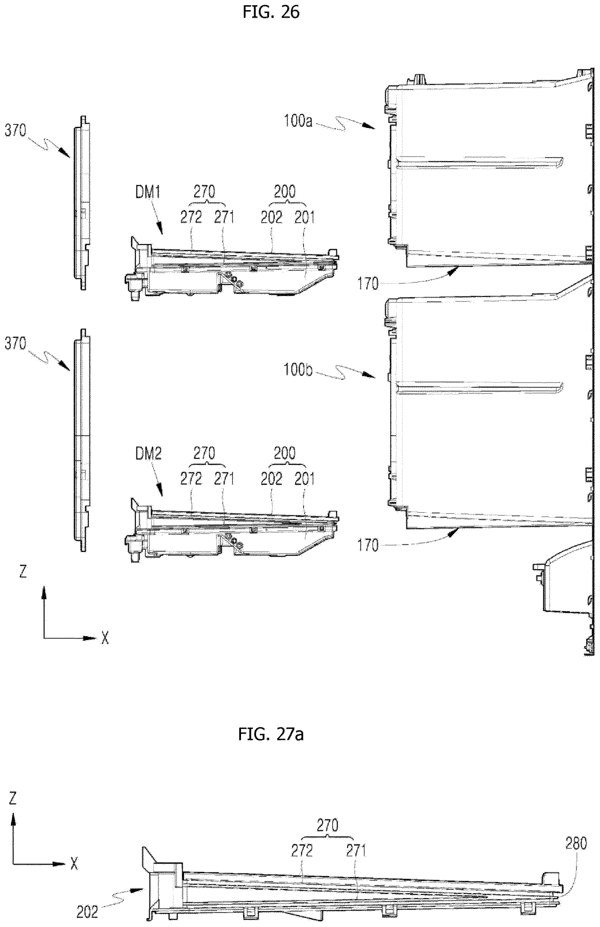

FIG. 26 is a side view illustrating a state in which the inner cabinets 100a and 100b, the module housing 200, and a drying air duct 370 are separated from each other in the shoes care device 1 according to one embodiment of the present invention.

FIG. 27a is a side view illustrating the module cover 202 according to one embodiment of the present invention.

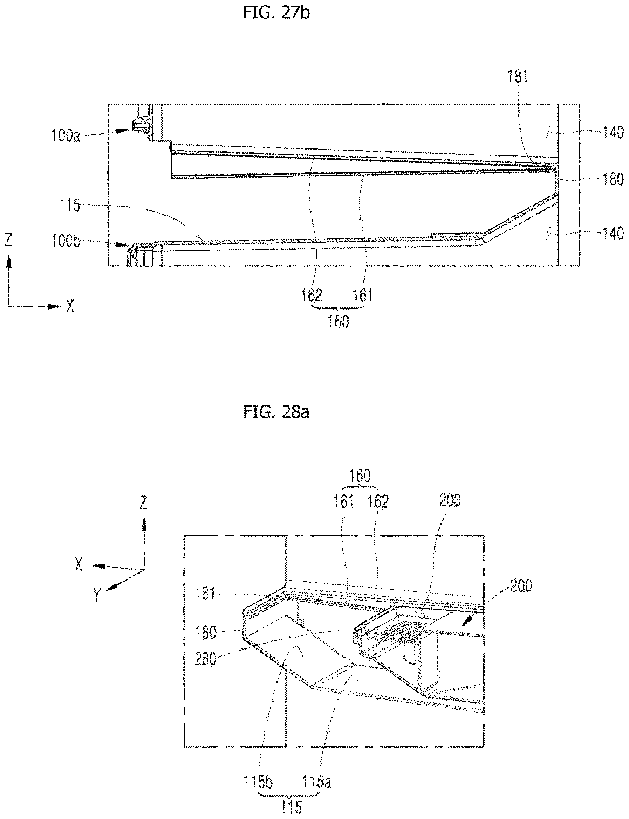

FIG. 27b is a cross-sectional view illustrating the inner cabinets 100a and 100b according to one embodiment of the present invention.

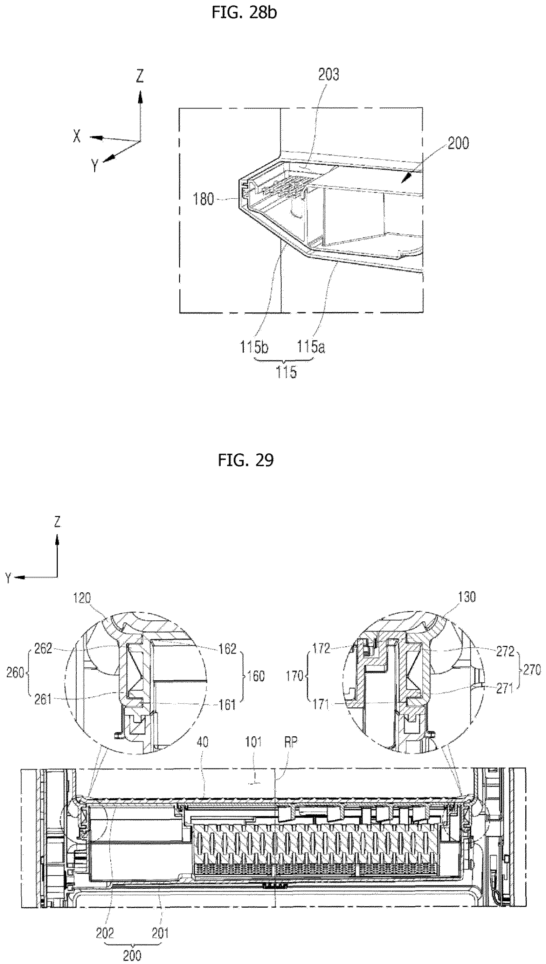

FIG. 28a is a cross-sectional perspective view of a part of the shores care device 1 before the coupling of the module housing 200 and the inner cabinet 100 is completed, and FIG. 28b is a cross-sectional perspective view of a part of the shores care device 1 where the coupling of the module housing 200 and the inner cabinet 100 is completed.

FIG. 29 is a cross-sectional view illustrating a state in which the inner cabinet 100 and the module housing 200 are coupled to each other in the shores care device 1 according to one embodiment of the present invention.

The sliding guides 160 and 170 are formed in the first direction (X direction) as the horizontal direction. The sliding guides 160 and 170 may be formed integrally with the inner cabinet 100. In the shoes care device 1 according to one embodiment of the present invention, the inner cabinet 100 may be formed by injection molding, and the inner cabinet 100 including the sliding guides 160 and 170 may be integrally formed by injection molding.

For injection molding of an injection-molded article with an undercut, when separating the injection-molded article from the mold, the injection-molded article may be elastically deformed and separated, a plurality of divided molds moving in different directions may be included, or a sliding core may be provided in the mold.

When the inner cabinet 100 is integrally molded by injection molding, when the undercut (an outer undercut or an inner undercut) is formed in the structure of the inner cabinet 100, manufacturing costs may increase or injection molding may be difficult.

In the shoes care device 1 according to one embodiment of the present invention, the inner cabinet 100 has a structure having no undercut or a structure with a minimized undercut structure. Accordingly, the inner cabinet 100 can be easily manufactured by injection molding, and further, the inner cabinet 100a of the first management device 2a and the inner cabinet 100b of the second management device 2b can be integrally formed.

When the inner cabinets 100a and 100b according to one embodiment of the present invention are manufactured by injection molding, a first mold IM1 having a shape corresponding to outer surfaces of the inner cabinets 100a and 100b and a second mold IM2 having a shape corresponding to inner surfaces of the inner cabinets 100a and 100b may be used.

The first mold IM1 and the second mold IM2 are closed, and then a molding material is supplied to a cavity CV between the first mold IM1 and the second mold IM2 and is injection-molded. Then, the injection-molded article (the inner cabinet 100) can be separated from the first mold IM1 and the second mold IM2 while opening the first mold IM1 and the second mold IM2.

Since the inner cabinets 100a and 100b have a main opening 140 opened in the first direction (X direction), the first mold IM1 and the second mold IM2 may be moved relative to each other in a direction parallel to the first direction (X direction).

Since the sliding guides 160 and 170 are formed in the inner cabinet 100 in the first direction (X direction), which is a formation direction of the main opening 140, the undercut can be excluded when the sliding guides 160 and 170 are formed. Accordingly, the sliding guide 100 may be easily formed in an injection-molded manner, and the first inner cabinet 100a of the first management device 2a and the second inner cabinet 100b of the second management device 2b may be integrally formed (see FIG. 20).

The sliders 260 and 270 may be fixed to the module case 201 or the module cover 202. The sliders 260 and 270 may be formed integrally with the module case 201 or the module cover 202.

As shown in the drawing according to one embodiment of the present invention, when the sliders 260 and 270 are formed integrally with the module cover 202, since the module cover 202 is located above the module case 201, the inner cabinet 100 and the module housing 200 may be more easily and stably coupled. In the shoes care device 1 according to one embodiment of the present invention, the module cover 202 may be formed by injection molding, and the module cover 202 including the sliders 260 and 270 may be integrally formed by injection molding.

The sliders 260 and 270 may be formed on both left and right edges of the module cover 202 with respect to the first direction (X direction), respectively. That is, a pair of sliders 260 and 270 may be provided in the module cover 202. The slider 260 formed on the left side of the module cover 202 and the slider 270 formed on the right side of the module cover 202 can be symmetrical to each other with respect to the reference plane RP.

The sliding guides 160 and 170 may be formed on left and right sides in the first direction (X direction) below the inner cabinet 100. That is, a pair of sliding guides 160 and 170 may be provided in the inner cabinet 100. In this case, the sliding guide 160 formed on the left side of the inner cabinet 100 and the sliding guide 170 formed on the right side of the inner cabinet 100 may be symmetrical to each other with respect to the reference plane RP.

The sliding guides 160 and 170 may be formed to extend from the rear to the front side of the inner cabinet 100. The sliding guides 160 and 170 may include lower rails 161 and 171 and upper rails 162 and 172.

The lower rails 161 and 171 are formed in a form parallel to or upwardly inclined along the first direction (X direction). The lower rails 161 and 171 may have a straight line shape.

The upper rails 162 and 172 are located above the lower rails 161 and 171 and are inclined downwards in the first direction (X direction). The upper rails 162 and 172 may have a straight line shape.

As the lower rails 161 and 171 and the upper rails 162 and 172 are formed in this way, the undercut can be excluded when the lower rails 161 and 171 and the upper rails 162 and 172 are molded, and the sliding guides 160 and 170 may easily injection-molding the integrally formed inner cabinet 100.

The sliding guides 160 and 170 in which the lower rails 161 and 171 and the upper rails 162 and 172 are combined, may have a tapered shape in which the vertical width thereof gets narrow toward the front side in the first direction (X direction). This structure of the sliding guides 160 and 170 makes it easier to open the mold IM1 and IM2 and separate the injection-molded article (the inner cabinet 100) from the mold IM1 and IM2 when the inner cabinet 100 is molded by injection molding.

When the sliding guides 160 and 170 are formed on the left and right sides under the inner cabinet 100 in the first direction (X direction), respectively, as described above, the sliders 260 and 270 are also formed on the left and right sides of the module housing 200 in the first direction (X direction), respectively.

The sliders 260 and 270 may be formed integrally with the module cover 202, and in this case, the sliders 260 and 270 may be formed to extend from the rear to the front of the module cover 202. The sliders 260 and 270 is configured to include lower sliders 261 and 271 and upper sliders 262 and 272.

The sliders 260 and 270 in which the lower sliders 261 and 271 and the upper sliders 262 and 272 are combined may have a tapered shape in which the vertical width thereof gets narrow toward the front side in the first direction (X direction).

The lower sliders 261 and 271 may be formed parallel to the lower rails 161 and 171. The lower sliders 261 and 271 may have a straight line shape. The lower sliders 261 and 271 may be in contact with the upper sides of the lower rails 161 and 171 and may slide in the first direction (X direction) or a direction opposite to the first direction (X direction).

The upper sliders 262 and 272 may be formed parallel to the upper rails 162 and 172. The upper sliders 262 and 272 may have a straight line shape. When the module housing 200 including the module cover 202 moves to the frontmost of the first direction (X direction) and when the coupling of the inner cabinet 100 and the module housing 200 is completed, the upper sliders 262 and 272 are in contact with the lower side of the upper rails 162 and 172. That is, before the coupling is completed by locating the module housing 200 behind the inner cabinet 100, the upper sliders 262 and 272 are spaced apart from the upper rails 162 and 172.

The sliders 260 and 270 are slidably coupled along the sliding guides 160 and 170, and when the module housing 200 is completely coupled to the inner cabinet 100, the module cover 202 completely shields the lower opening 150 of the inner cabinet 100.

As described above, in one inner cabinet 100, a pair of sliding guides 160 and 170 may be provided and formed on both left and right sides of the inner cabinet 100. In this case, the pair of sliding guides 160 and 170 may be divided into a first sliding guide 160 and a second sliding guide 170.

The first sliding guide 160 may be formed in a lower end of the first inner side plate 120 in the first direction (X direction). The second sliding guide 170 may be formed in a lower end of the second inner side plate 130 in the first direction (X direction).

In addition, the lower opening 150 of the inner cabinet 100 has a hole shape opened between the first sliding guide 160 and the second sliding guide 170.

In addition, as described above, a pair of sliders 260 and 270 may be provided and formed on both left and right sides of the module housing 200 (the module cover 202). In this case, the pair of sliders 260 and 270 may be divided into a first slider 260 and a second slider 270.

The first sliding guide 160 and the second sliding guide 170 are symmetrical with respect to the reference plane RP, and the first slider 260 and the second slider 270 are symmetrical with respect to the reference plane RP.

In the shoes care device 1 according to one embodiment of the present invention, when the inner cabinets 100a and 100b and the module housing 200 are coupled, if the module housing 200 moves to the frontmost part in the first direction (X direction), the upper rails 162 and 172 and the upper sliders 262 and 272 come into contact with each other, and the upper rails 162 and 172 serve as stoppers. Therefore, with the movement of the module housing 200 in the first direction (X direction), stable assembly between the module housing 200 and the inner cabinet 100 is achieved, and vertical and horizontal gaps between the module housing 200 and the inner cabinet 100 are effectively prevented.

Each of the sliding guides 160 and 170 and the sliders 260 and 270 is provided in pairs and is symmetrically formed on both left and right sides of the inner cabinet 100 with respect to the first direction (X direction), thereby stably and easily coupling and separation of the module housing 200 and the inner cabinet 100.

The inner cabinet 100 includes a front frame 180 and a fixing guide 181. The front frame 180 and the fixing guide 181 may be formed integrally with the inner cabinet 100.

The front frame 180 is formed in the inner cabinet 100 ahead in the first direction (X direction). The front frame 180 may be bent outwards from the front end of the inner cabinet 100 with respect to the first direction (X direction). The front frame 180 may form a surface orthogonal to the first direction (X direction). The front surface of the front frame 180 may be orthogonal to the first direction (X direction).

The front frame 180 is configured to shield the front end of the module housing 200 with respect to the first direction (X direction).

The front frame 180 connects a front end of the first inner side plate 120 and a front end of the second inner side plate 130 to each other. The front frame 180 connects a lower side of the front end of the first inner side plate 120 and a lower side of the front end of the second inner side plate 130 to each other. The front frame 180 may be formed along the entire frame of the inner cabinet 100 ahead in the first direction (X direction), and the area surrounded by the front frame 180 corresponds to the main opening 140.

The inner cabinet 100a of the first management device 2a and the inner cabinet 100b of the second management device 2b are connected to each of front frames 180. When the first inner cabinet 100a and the second inner cabinet 100b are integrally formed by injection molding, the front frame 180 of the first inner cabinet 100a and the front frame 180 of the second inner cabinet 100b are formed integrally with each other.

The front frame 180 of the second inner cabinet 100b may be formed integrally with the first wall 51.

The fixing guide 181 is formed on a rear surface of the front frame 180 with respect to the first direction (X direction). The fixing guide 181 may protrude to the opposite side of the first direction (X direction). The fixing guide 181 may be formed in a shape extending in the second direction (Y direction). The fixing guide 181 may have a constant cross-section in the second direction (Y direction).

The module cover 202 includes a fixing rib 280.

The fixing rib 280 may protrude from the front end of the module cover 202 in the first direction (X direction) to the first direction (X direction). Further, the fixing rib 280 may be formed to extend in the second direction (Y direction). The fixing rib 280 may have a constant cross-section in the second direction (Y direction).

The fixing guide 181 and the fixing rib 280 may be engaged with each other. When one of the fixing guide 181 and the fixing rib 280 is formed in a protrusion shape, the other may be formed in a groove shape into which the protrusion is inserted. When the fixing rib 280 is formed in a protrusion shape protruding in the first direction (X direction), the fixing guide 181 may be formed in a groove shape that is concave in the first direction (X direction), and the fixing rib 280 may be inserted into the fixing guide 181 and be coupled to each other.

In one embodiment of the present invention, when the sliders 260 and 270 of the module housing 200 move in the first direction (X direction) along the sliding guides 160 and 170 of the inner cabinet 100, and the coupling of the module housing 200 and the inner cabinet 100 is completed, the coupling the fixing rib 280 and the fixing guide 181 may are made to be completed.

In the shoes care device 1 according to one embodiment of the present invention, when the door 30 is opened, the inner cabinet 100 is exposed from the frontmost part of the front frame 180, and there is no need to form any division line (e.g., a division line where the inner cabinets 100 are coupled to each other) in the front frame 180. Accordingly, the shoes care device 1 can be form which is a structurally stable and has excellent aesthetics.

The inner cabinet 100 includes an inner upper plate 115 forming a ceiling.

The inner cabinet 100b of the second management device 2b may include the inner upper plate 115 which forms the ceiling and is connected to the front frame 180, where a front part thereof in the first direction (X direction) forms an upwardly inclined surface.

The inner upper plate 115 of the inner cabinet 100 may include a first region 115a and a second region 115b. In each of the inner cabinet 100a of the first management device 2a and the inner cabinet 100b of the second management device 2b, the inner upper plate 115 may include the first region 115a and the second region 115b.

In the first direction (X direction), the first region 115a forms a relatively rear part and a middle part, and the second region 115b forms a relatively front part.

The first region 115a forms a flat surface. The first region 115a may form a substantial part of the inner upper plate 115.

In one embodiment, the first region 115a may be parallel to the horizontal direction.

In another embodiment, the first region 115a may be inclined upwards in the first direction (X direction). The first region 115a may be formed smaller than an inclination angle formed by the second region 115b and inclined upwards in the first direction (X direction).

The second region 115b extends ahead from the front of the first region 115a with respect to the first direction (X direction). The second region 115b forms a surface upwardly inclined in the first direction (X direction) and forms a region connected to the front frame 180.

The second region 115b of the second management device 2b may have a greater inclination angle formed by a horizontal surface than the second region 115b of the first management device 2a.

Accordingly, even if the width of the front frame 180 is not increased when viewed from the front, a space in which the module housing 200 is coupled to the lower side of the inner cabinet 100 can be secured in the first management device 2a.

In addition, the structure of the inner upper plate 115 including the first region 115a and the second region 115b does not act as an undercut when the inner cabinet 100 is molded by injection molding, and is easily used to open the molds IM1 and IM2 and separate the injection-molded article (the inner cabinet 100) from the molds IM1 and IM2).

The front frame 180 of the first inner cabinet 100a may be connected to the inner upper plate 115 of the second inner cabinet 100b. The front frame 180 of the first inner cabinet 100a and the inner upper plate 115 of the second inner cabinet 100b may be connected to each other in the entire section in the second direction (Y direction).

The first inner side plate 120 may extend downwards from one end of the inner upper plate 115, and the second inner side plate 130 may extend downwards from the other end of the inner upper plate 115.

In one embodiment, the first inner side plate 120 and the second inner side plate 130 may be parallel to each other in the first direction (X direction).

In another embodiment, the first inner side plate 120 and the second inner side plate 130 may be inclined in a direction moving away from each other along the first direction (X). In one embodiment, an angle formed between the first inner side plate 120 and a vertical surface and an angle formed between the second inner side plate 130 and the vertical surface may be 5 degrees or less, respectively.

Each of the first inner side plate 120 and the second inner side plate 130 includes a shelf holder 910 protruding inwards from the inner side and formed in the first direction (X direction).

The shelf holder 910 may be formed such that the vertical width ht becomes narrower toward the first direction (X direction).

The shelf holder 910 formed on the first inner side plate 120 and the shelf holder 910 formed on the second inner side plate 130 are formed integrally with the inner cabinet 100.

Since the shelf holder (910) is formed parallel to the first direction (X direction) and the vertical widths ht thereof becomes narrower toward the first direction (X direction), the shelf holder 910 does not act as an undercut when the inner cabinet 100 is injection-molded, this makes it easy to open the molds IM1 and IM2 and separate the inner cabinet 100 from the molds IM1 and IM2.

As described above, the first management device 2a and the second management device 2b may be vertically disposed, and in this case, the first inner cabinet 100a and the second inner cabinet 100b may be formed integrally with each other. Accordingly, no separate process to couple the first inner cabinet 100a and the second inner cabinet 100b is required, the sense of unity between the first inner cabinet 100a and the second inner cabinet 100b can be improved, and the overall aesthetics and productivity of the shoes care device 1 can be improved.

In the shoes care device 1 according to one embodiment of the present invention, the inner cabinet 100 of the first management device 2a is disposed above the inner cabinet 100 of the second management device 2b. The hoes care device 1 may be formed where the inner cabinet 100 and the module housing 200 of the first management device 2a, and the inner cabinet 100 and the module housing 200 of the second management device 2b are arranged in order from top to bottom, and the shoes care device 1 may be formed which can utilize a space in the vertical direction. In this case, the module housing 200 is coupled to a lower side of the inner cabinet 100 while sliding in the first direction (X direction). In other words, the module housing 200 of the first management device 2a is slidably coupled between the first inner cabinet 100a and the second inner cabinet 100b, and the module housing 200 of the second management device 2b is slidably coupled to the lower end of the second inner cabinet 100b.

The structure of coupling the inner cabinet 100 and the module housing 200 allows a plurality of inner cabinets 100 to be integrally formed by injection molding, and allows a stable coupling between the inner cabinet 100 and the module housing 200 without wasting a space.

As the module housing 200 slides in the first direction (X direction) and is coupled to the inner cabinet 100, the inner cabinet 100 and the module housing 200 can be easily and stably coupled to each other. In addition, when the inner cabinet 100 or its internal components need to be repaired or replaced, the module housing 200 can be easily separated from the inner cabinet 100 by moving the module housing 200 behind the first direction (X direction).

In the shoes care device 1 according to one embodiment of the present invention, the module cover 202 constituting the module housing 200 is provided an inlet 203 formed through which air of the accommodation space 101 is sucked, and the inner cabinet 100 includes a lower opening 150 formed by opening an upper part thereof. When the module housing 200 is coupled to the inner cabinet 100, the module cover 202 is formed to close the lower opening 150. That is, the module cover 202 of the module housing 200 forms a bottom surface of the inner cabinet 100.

When a complex structure is required on the bottom of the inner cabinet 100, unlike the embodiment of the present invention, assuming that the entire inner cabinet 100 including the bottom of the inner cabinet 100 is formed by injection molding, manufacturing costs may increase or manufacturing thereof may be difficult. This is because the structure of the bottom of the inner cabinet 100 and the outlet 203 act as undercuts. When the first inner cabinet 100a and the second inner cabinet 100b are arranged vertically and integrally with each other, such difficulties may be further increased.

Unlike the embodiment of the present invention, assuming that the module cover 202 is formed integrally with the inner cabinet 100 and the inner cabinet 100 is formed by injection molding, the molds (the first mold IM1 and the second mold IM2) for manufacturing the inner cabinet 100 needs to mold the inner cabinet 100 while being closed and opened with a relatively movement in a direction in parallel with the first direction (X direction), but there is any difficulty forming what structure is formed on the bottom of the module cover 202, and also forming a part of the outlet 203 vertically penetrating the module cover 202. In particular, it is more difficult to form the inner cabinet 100 of the first management device 2a and the inner cabinet 100 of the second management device 2b integrally by injection molding.

As described above, in the shoes care device 1 according to one embodiment of the present invention, as the module cover 202 of the module housing 200 formed separately from the inner cabinet 100 forms the bottom surface of the inner cabinet 100, the module cover 202 may be easily formed even when a relatively complex structure for forming a connection path is required on the lower surface of the module cover 202, the inner cabinet 100 may also be easily formed by injection molding, and then, by coupling the module housing 200 to the inner cabinet 100, the bottom surface of the inner cabinet 100 may be easily formed.

Even when the first inner cabinet 100a and the second inner cabinet 100b are vertically arranged and integrally formed, the inner cabinet 100a and 100b may be first manufactured by injection molding and then the inner cabinet 100a and 100b may be coupled to the module housing 200, thereby easily forming the bottom of the inner cabinet 100a and 100b.

In addition, the inner upper plate 115, the first inner side plate 120, and the second inner side plate 130 are formed in the first direction (X direction), which is the formation direction of the main opening 140, the sliding guides 160 and 170 formed integrally with the inner cabinets 100a and 100b and the shelf holder 910 are formed in the first direction (X direction), and the inner upper plate 115, the first inner side plate 120, the second inner side plate 130, the sliding guides 160 and 170, and the shelf holder 910 may all be formed without undercut based on the first mold IM1 and the second mold IM2 that move relative to each other in the first direction (X direction). Accordingly, the inner cabinet 100 may be integrally manufactured by injection molding.

In the shoes care device 1 according to one embodiment of the present invention, the inner cabinet 100 of the first management device 2a and the inner cabinet 100 of the second management device 2b may be integrally formed by injection molding to form the shoes care device 1 with a structure advantageous for rigidity. In addition, it is possible to reduce the components necessary for manufacturing the shoes care device 1 and reduce material costs and manufacturing costs.

In addition, the front part of the module housing 200 is covered by the front frame 180 and the front frame 180 integrated with the inner cabinet 100 is exposed from the front when the door 30 is opened. Accordingly, the module housing 200 and the inner cabinet 100 can be integrally coupled to each other, and the shoes care device 1 with an excellent esthetic sense on the whole can be formed.

The shoes care device 1 according to one embodiment of the present invention includes a dry air duct 370 that connects the module housing 200 and the inner cabinet 100 in communication with each other. In the module cover 202, the outlet 203 is formed in the front side in the first direction (X direction), and the dry air duct 370 connects the module housing 200 and the inner cabinet 100 in communication with each other in the rear side in the first direction (X direction). Accordingly, it is possible to easily form a shoes care device 1 with a structure in which the module housing 200 can be first coupled to the inner cabinet 100 and the dry air duct 370 can be coupled to the inner cabinet 100 and the inside of the module housing 200, and air can be circulated inside the inner cabinet 100 and inside the module housing 200.

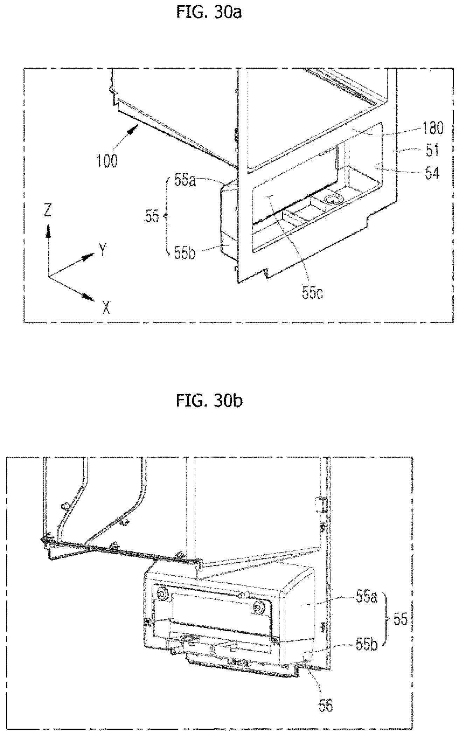

FIG. 30a is a perspective view illustrating the lower side of the inner cabinet 100 and the first wall 51, and FIG. 30b is a rear perspective view illustrating the lower side of the inner cabinet 100 and the first wall 51.

A water tank opening 54, which is a hole penetrated in the first direction (X direction), is formed in the first wall 51.

Each of the water supply tank 60 and the drain tank 70 may be disposed in the water tank opening 54. When the water supply tank 60 and the drain tank 70 are disposed in the water tank opening 54, the water tank opening 54 may be shielded by the water supply tank 60 and the drain tank 70.

The front frame 180 and the first wall 51 may form the same plane and be formed integrally with each other.

The shoes care device 1 may include an upper accommodation housing 55a.

The upper accommodation housing 55a is integrally connected to the first wall 51 in an edge of the water tank opening 54. The upper accommodation housing 55a extends behind the first wall 51 with respect to the first direction (X direction), and forms a water tank accommodation space 55c for accommodating the water supply tank 60 and the drain tank 70.

The upper part of the upper accommodation housing 55a is inclined downwardly toward the direction opposite to the first direction (X direction). The upper accommodation housing 55a is formed to cover the upper sides of the water supply tank 60 and the drain tank 70.

The shoes care device 1 includes a lower accommodation housing 55b.

The lower accommodation housing 55b is formed to cover the lower sides of the water supply tank 60 and the drain tank 70. The lower accommodation housing 55b and the upper accommodation housing 55a are coupled to form the accommodation housing 55. The lower accommodation housing 55b is coupled to a lower side of the upper accommodation housing 55a to form the water tank accommodation space 55c along with the upper accommodation housing 55a.

By forming the first wall 51 and the upper accommodation housing 55a integrally with the front frame 180 of the inner cabinet 100, a structurally stable and aesthetic shoes care device 1 can be formed, and the water supply tank 60 and the drain tank 70 can be stably coupled with the upper accommodation housing 55a.

Since the upper part of the upper accommodation housing 55a is inclined downwards in the direction opposite to the first direction (X direction), even if the width of the front frame 180 of the inner cabinet 100 of the second management device 2b is not increased, a space in which the module housing 200 is coupled to the lower side of the inner cabinet 100 of the second management device 2b may be secured. In addition, the inner cabinet 100 may be easily manufactured by injection molding.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### [Industrial Applicability]

According to the embodiment, a shoes care device having excellent productivity and use convenience can be provided.

## Claims

1. A shoes care device comprising:
an inner cabinet having an accommodation space accommodating shoes;
a module housing coupled to a lower side of the inner cabinet, and having a module chamber communicating with the accommodation space therein;
a sump provided at the lower side of the module housing and configured to accommodate condensed water;
a blowing part accommodated in the module chamber, and blowing air of the module chamber;
a heating part accommodated in the module chamber, and heating the air of the module chamber;
a dehumidifying part accommodated in the module chamber, and dehumidifying the air of the module chamber;
a drying flow path forming a path so as for the air of the module chamber passing through the dehumidifying part to be re-introduced into the accommodation space; and
a regeneration path forming a path so as for the air of the module chamber passing through the dehumidifying part to move to the sump,
wherein the module housing is detachably coupled to the lower side of the inner cabinet.

2. The shoes care device of claim 1, wherein the module housing is coupled to the lower side of the inner cabinet while sliding in a horizontal direction.

3. The shoes care device of claim 1, wherein the inner cabinet includes a sliding guide formed in a first direction which is a horizontal direction, and
the module housing includes
a module case forming the module chamber, and having a module opening opened upward, and having an area on a plan view, which is equal to or larger than an area of the module chamber,
a module cover covering the module opening at the upper side of the blowing part, the heating part and the dehumidifying part, and coupled to the module case, and having an outlet through which the air of the accommodation space is suctioned to the module chamber, which is penetrated, and
a slider fixed to the module case or the module cover, and configured to slidably move in a forming direction of the sliding guide.

4. The shoes care device of claim 3, wherein the sliding guide includes
a lower rail parallel or inclined upward along the first direction, and
an upper rail positioned at the upper side of the lower rail, and inclined downward in the first direction, and
the slider includes
a lower slider contacting the upper side of the lower rail and parallel to the lower rail, and
an upper slider parallel to the upper rail, and contacting the lower side of the upper rail when the module housing moves at a frontmost portion of the first direction.

5. The shoes care device of claim 3, wherein the inner cabinet includes a main opening of which a front side of the first direction is opened,
the shoes care device includes a door that opens/closes the main opening,
a pair of sliding guides are provided, and formed to be symmetric to both left and right sides of the inner cabinet based on the first direction, and
a pair of sliders are provided, and formed to be symmetric to both left and right sides of the module housing based on the first direction.

6. The shoes care device of claim 3, wherein the outlet is formed in a second direction which is orthogonal to the first direction and which is a horizontal direction at the front of the module cover based on the first direction, and
an upper surface of the module cover is inclined downward in the first direction.

7. The shoes care device of claim 6, wherein the inner cabinet includes a lower opening of which a lower portion is opened, and
when the module housing is coupled to the inner cabinet, the module cover closes the lower opening.

8. The shoes care device of claim 1, wherein the module housing includes
a module case forming the module chamber, and having a module opening opened upward, and
a module cover covering the module opening at the upper side of the blowing part, the heating part and the dehumidifying part, and coupled to the module case, and having an outlet through which the air of the accommodation space is suctioned to the module chamber, which is formed to penetrate, and
the module cover is coupled to a lower end of the inner cabinet to slidably move in the horizontal direction,
two or more inner cabinets, module housings, blowing parts, heating parts, and dehumidifying parts are each provided, and
two or more inner cabinets are configured integrally with each other while being placed vertically.

9. The shoes care device of claim 8, wherein the inner cabinet includes
a first inner side plate positioned at any one based on a reference plane which is parallel to the first direction which is the horizontal direction, and which is a vertical surface,
a second inner side plate positioned at an opposite side to the first inner side plate based on the reference plane,
a first sliding guide formed in the first direction at a lower end of the first inner side plate,
a second sliding guide formed in the first direction at a lower end of the second inner side plate, and
a lower opening which is a hole opened between the first sliding guide and the second sliding guide, and
wherein the module cover includes
a first slider coupled to the first sliding guide to be slidably movable, and
a second slider coupled to the second sliding guide to be slidably movable.

10. The shoes care device of claim 9, wherein when the first direction is a forward direction,
the inner cabinet includes
a front frame connecting a front of the first inner side plate and a front of the second inner side plate to each other, and
a fixing guide formed on a back surface of the front frame in a second direction which is orthogonal to the first direction and which is the horizontal direction,
the module cover includes a fixation rib formed in the second direction,
the fixing guide and the fixation rib are engaged with each other.

11. The shoes care device of claim 9, wherein the first sliding guide and the second sliding guide are symmetric to each other based on the reference plane, and
the first slider and the second slider are symmetric to each other based on the reference plane.

12. The shoes care device of claim 3, wherein the outlet through which the air of the accommodation space is suctioned to the module chamber is formed to penetrate at the front of the first direction in the module cover, and
the shoes care device includes a dry air duct connecting the module housing and the inner cabinet to be in communication with each other at a rear of the first direction, and forming the entirety or a part of the drying flow path.

13. The shoes care device of claim 12, wherein the module housing includes a dry air outlet opened at a rear side of the module housing based on the first direction, and
the dry air duct is closely coupled to the module housing and the inner cabinet while covering the dry air outlet.

14. The shoes care device of claim 12, wherein the module housing includes a steam inlet provided to be in communication with the accommodation space at the rear of the first direction, and
the shoes care device includes
a steam generator provided at the lower side of the module housing, and generating a steam, and
a steam separator including a separating base coupled to the module housing at the rear of the first direction and forming an internal space which is in communication with the steam inlet, a separating inlet formed at one side of the separating base and connected to the steam generator, and a separating outlet formed at the lower side of the separating base and discharging condensed water condensed inside the separating base.

15. The shoes care device of claim 2, wherein the module chamber includes
a first module chamber accommodating the blowing part,
a second module chamber accommodating the heating part, and
a third module chamber accommodating the dehumidifying part, and
the first module chamber, the second module chamber, and the third module chamber are formed at different locations on a plan view.

16. The shoes care device of claim 15, wherein the air of the module chamber moves to the first module chamber, the second module chamber, and the third module chamber sequentially.

17. The shoes care device of claim 16, wherein the module housing includes
a module case forming the module chamber, the module case including a suction module chamber which is a part of the module chamber, a dry air outlet opened so as for the air of the third module chamber to be discharged and connected to the accommodation space, and a wet air outlet opened so as for the air of the third module chamber to be discharged and connected to the sump, and
a module cover covering the upper side of the blowing part, the heating part, and the dehumidifying part and coupled to the module case, and having an outlet through which the air of the accommodation space is suctioned to the module chamber, which is formed to penetrate at the upper side of the suction module chamber, and
when a direction in which the module housing is coupled at the lower side of the inner cabinet is the first direction,
the suction module chamber, the first module chamber, the third module chamber, and the dry air outlet are placed in order toward the rear from the front based on the first direction, and
a bottom surface of the suction module chamber is inclined downward toward the first module chamber.

18. The shoes care device of claim 17, wherein the shoes care device includes
a damper closing the dry air outlet or the wet air outlet, and
a condenser connected to be in communication between the wet air outlet and the sump, forming the entirety or a part of the regeneration path, and coupled to an outer surface of the inner cabinet.

19. The shoes care device of claim 16, wherein the blowing part is spaced apart from a bottom of the first module chamber so that the air is introduced into the inside of the blowing part from the lower side of the first module chamber inside the first module chamber.

20. The shoes care device of claim 16, wherein the dehumidifying part is spaced apart from the bottom of the third module chamber so that the air moves to the lower side by penetrating the dehumidifying part from the upper side of the third module chamber inside the third module chamber.
